(19) Europäisches Patentamt
European Patent Office
Office européen des brevets

(11) Publication number: 0 254 413 A2

(12) # EUROPEAN PATENT APPLICATION

(21) Application number: 87305270.8

(22) Date of filing: 12.06.87

(51) Int. Cl.[3]: A 61 K 33/38
A 01 N 59/16

(30) Priority: 13.06.86 JP 137786/86
29.08.86 JP 203472/86
29.08.86 JP 203473/86
14.10.86 JP 243656/86
14.10.86 JP 243657/86
14.10.86 JP 243658/86
28.10.86 JP 256389/86

(43) Date of publication of application:
27.01.88 Bulletin 88/4

(84) Designated Contracting States:
DE FR GB IT

(71) Applicant: Matsuo, Yoshiaki
2-19-11, Omorihoncho Ota-ku
Tokyo(JP)

(71) Applicant: Ito, Jin-ichi
1-2-1, Nishiwaseda Shinjuku-ku
Tokyo(JP)

(71) Applicant: Oshima, Katsue
4-26-9, Shichirigahamahigashi
Kamakura-shi Kanagawa-ken(JP)

(72) Inventor: Matsuo, Yoshiaki
2-19-11 Omorihoncho Ota-ku
Tokyo(JP)

(72) Inventor: Oshima, Katsue
4-26-9 Shichirigahamahigashi
Kamakura-shi Kanagawa-ken(JP)

(72) Inventor: Ito, Jin-Ichi
1-2-1 Nishiwaseda Shinjuku-ku
Tokyo(JP)

(72) Inventor: Yamamoto, Sugi
1-14-38 Minamisawa
Higashikurume-shi Tokyo(JP)

(74) Representative: Skailes, Humphrey John et al,
Frank B. Dehn & Co. Imperial House 15-19 Kingsway
London WC2B 6UZ(GB)

(54) **Silver-ionic water and its uses.**

(57) A silver-ionic water having the pH of not more than 5 or not less than 8 and silver ion concentration of 0.03 to 10 ppm. The silver-ionic water can be used as a bacteriostatic water, a growth promotive acidic water for plant cultivation, a growth promotive alkaline water for plant cultivation, a therapeutic water for pathogenobacterial skin diseases and external cavity mucosal diseases, a wound-curative water, a disinfectant water, or a therapeutic water for digestive organ mucosal diseases and keratoconjunctivitis.

EP 0 254 413 A2

# SILVER-IONIC WATER AND ITS USES

## BACKGROUND OF THE INVENTION

This invention relates to a silver-ionic water (or silver ion-containing water) used in the field of, for example, sanitary administration in processing, distribution and selling of food, soil conditioning and cultivation promotion in agriculture, environmental sanitation in medical care, etc.

Silver-ionic water, which is less dangerous for human bodies and has an bactericidal action, has recently attracted notices as potable water, sterilized water and so forth.

In the present invention, the action expected in silver ions include a bacteriostatic action against bacteria, an action of supplying activated oxygen to roots of plants, an action of supplying OH ions for the purpose

of promoting photosynthesis in the cells of plants with a lower amount of light, and action such as elimination of pains, hemostasis and promotion of a vulnerary action. Of these, the bacteriostatic action originates from the oxidation catalytic function inherent in silver acting on a cell membrane of bacteria, the silver concentration, and the pH of ionic water. Upon contact of silver ions with a bacterial body, the catalytic function of silver may cause a radical reaction in the unsaturated aliphatic acid constituting a hydrophobic part of a cell membrane of bacteria to hydrolyze the same. The cell membrane of bacteria thereby loses its function, aiming at stopping vital activities by inflow of water, and DNA and constituents of a nuclear membrane may not be affected by the catalytic function of silver to remain in a sound form. Moreover, silver tends to be little accumulated inside a living body as compared with other metallic elements. From these facts, silver ions are considered to be little dangerous for human bodies and, from a genetic viewpoint also, cause no disorder in a fetus that may affect a next generation, and therefore, if conditions are adjusted, has a possibility to serve as a new bacteriostatic agent substituting conventional chemical medicaments.

Conventionally, the silver-ionic water has been

produced by an apparatus embodying a process called a Katadyn process. This apparatus comprises an anode and a cathode, wherein the anode is provided with silver, and water is allowed to pass while a voltage is applied between both electrodes, whereby silver is ionized and dissolved out to give silver-ionic water.

However, the apparatus mentioned above is involved in a problem that the resulting silver-ionic water is substantially neutral, and, under the neutral condition, silver ions may be adsorbed on proteins and can have only a little chance for direct contact with the inside of a cell of a bacterial body when proteins are contained in water or bacteria are in contact with proteins, resulting in an extreme lowering of its bacteriostatic action. Specifically speaking based on an experimental work made by the present inventors, this silver-ionic water was brought into contact with bacteria in an aqueous solution in which proteins are dissolved, and also, separately, with bacteria adhered to proteins, but, because of the neutral environment, metallic ions were combined to proteins, releasing ions, and was not able to enter the cells of bacteria when brought into contact with the bacteria, with the loss of the effect by silver ions and no achievement of the object.

To cope with the problem, the present inventors have

made intensive studies. As a result, they have found that silver-ionic water having a given silver ion concentration under the acidic condition of pH 5 or less or the alkaline condition of pH 8 or more may have a remarkable bacteriostatic action against bacteria present together with proteins, with a silver ion concentration that may be less dangerous for human bodies, and that the acidic silver-ionic water has an action of supplying activated oxygen to roots of plants and the alkaline silver-ionic water, when supplied to plant cells, has an action of promoting photosynthesis.

As an apparatus for producing acidic or alkaline silver-ionic water, known is an apparatus as disclosed in Japanese Unexamined Patent Publication No. 97088/1985, for example. This apparatus comprises an electrolytic cell provided with an anode comprising a silver electrode, and a cathode comprising a carbon electrode or the like, wherein a cylindrical ion-exchange diaphragm is disposed around the anode, which diaphragm defines an anode compartment and a cathode compartment which are separated from each other, and a feed back pipe through which the water is circulated from the cathode compartment to the anode compartment. In this apparatus, with application of voltage between both of the electrodes, acidic anionic water can be obtained from the anode compartment, and

alkaline silver anionic water, from the cathode compartment.

However, in the above apparatus, a high voltage have had to be applied in order to attain the desired EC value by applying a voltage to the anode 12 and the cathode 13. Therefore, because of the silver electrode provided on the anode 12, if it is attempted to increase the silver ion concentration of the water to be taken out, silver particles begins to precipitate to form colloids when the silver ion concentration reaches a certain level. Thus, there has been a limit in increasing the silver ion concentration. Moreover, there has been a fear of adversely affecting human bodies when silver grains are precipitated in a large quantity to form colloids.

Also, in the above apparatus, a great electric power is required to achieve the desired pH of pH 5 or less and pH 8 or more, so that silver particles may precipitate to form colloids of silver particles, and the silver-ionic water thus obtained can be of no use.

## SUMMARY OF THE INVENTION

An object of the present invention is to provide a silver-ionic water which can be acidic or alkaline and also have the desired silver ion concentration.

Another object of the present invention is to provide a variety of new uses of such silver-ionic water, as will be made apparent from the descriptions set out below.

According to the present invention, there is provided a silver-ionic water having the pH of 5 or less or the pH of 8 or more, and having a silver ion concentration of 0.03 to 10 ppm.

According to the present invention, there are also provided new uses of such silver-ionic water.

## BRIEF DESCRIPTION OF THE DRAWING

Fig. 1 is a partially cross-sectional view showing an example of the apparatus for producing the silver-ionic water of the present invention;

Fig. 2 is a graphic illustration of the results obtained by examining influences of salt concentration and pH to the combination of serum albumin with acetyl-L-tryptophan;

Fig. 3 is a graphic illustration of bacteriostatic action against Staphylococcus, of various kinds of the silver-ionic water prepared by varying the silver ion concentration and the pH;

Fig. 4 is a graphic illustration of bacteriostatic

action against Escherichia coli, of various kinds of the silver-ionic water prepared by varying the silver ion concentration and the pH;

Fig. 5 is a graphic illustration of bacteriostatic action against general various bacteria, of various kinds of the silver-ionic water prepared by varying the silver ion concentration and the pH;

Fig. 6 is a graphic illustration of the change in the number of bacteria in a soil to which the growth promotive acidic water of the present invention has been added;

Fig. 7 is a graphic illustration of bactericidal action against tobacco mosaic virus, of the growth promotive alkaline water of the present invention; and

Fig. 8 is a graphic illustration of bactericidal action by ultraviolet irradiation against tobacco mosaic virus.

## DESCRIPTION OF THE PREFERRED EMBODIMENTS

The silver-ionic water of the present invention can be obtained according to a process for producing silver-ionic water, comprising;

a step of passing water through a first electrolytic cell chamber containing an anode and a cathode and

provided with silver on the anode, under the application of a voltage, to dissolve out silver ions; and

a step of passing the water in which said silver ions have been dissolved out, through either one or both of an anode compartment and a cathode compartment of a second electrolytic cell chamber containing an anode and a cathode and separated into said anode compartment and said cathode compartment with a diaphragm formed between both of said electrodes.

As an apparatus for carrying out the above process, there may be used, for example, an apparatus for producing silver-ionic water, comprising;

a first electrolytic cell chamber containing an anode and a cathode and provided with silver on the anode,

a second electrolytic cell chamber containing an anode and a cathode and separated into an anode compartment and a cathode compartment with a diaphragm formed between both of said electrodes, and

at least one of a flow path in which water is passed from said first electrolytic cell chamber through the anode compartment of said second electrolytic cell chamber and flowed out, and a flow path in which water is passed from said first electrolytic cell chamber through the cathode compartment of said second electrolytic cell chamber and flowed out.

According to the above apparatus, acidic silver-ionic water such as $Ag^+$-containing water can be obtained by dissolving out silver ions in water in the first electrolytic cell chamber and passing this water through the anode compartment of the second electrolytic cell chamber, and alkaline silver-ionic water such as water containing $Ag(OH)_2^-$, $Ag_2(OH)^+$, etc. can be obtained by passing the water treated in the first electrolytic cell chamber through the cathode compartment of the second electrolytic cell chamber. Also, in the apparatus for carrying out this process, the voltage, current, and flow rate of water in the first electrolytic cell chamber may be controlled to attain a desired silver ion concentration, without formation of colloids of silver particles even when the silver ion concentration has been increased. This is because the desired silver ion concentration is controlled in the first electrolytic cell chamber and the water having been made silver-ionic is made acid or alkaline and made to have an increased EC value in the second electrolytic cell chamber. According to the apparatus of the present invention, it is also possible to produce an acidic silver-ionic water having an increased acidic side electrochemical potential and an alkaline silver-ionic water to have respectively the desired pH value, EC value and amount of dissolved oxygen

by controlling the voltage, current, and flow rate of water in the second electrolytic cell chamber.

According to a preferred embodiment of the above apparatus, there are provided, in addition to the above water flow paths, a flow path in which the water is directly passed though the above anode compartment of the second electrolytic cell chamber and flowed out, and another flow path in which the water is directly passed through the above cathode compartment of the second electrolytic cell chamber and flowed out. According to this embodiment, water is passed from the first electrolytic cell chamber to the second electrolytic cell chamber and at the same time another water is directly passed through the cathode compartment of the second electrolytic cell chamber, so that, while controlling the flow rate of both the water, desired acidic silver-ionic water only can be taken out from the anode compartment of the second electrolytic cell chamber. Alternatively, water is passed from the first electrolytic cell chamber to the anode compartment of the second electrolytic cell chamber and at the same time another water is directly passed through the anode compartment of the second electrolytic cell chamber, so that, while controlling the flow rate of both the water, desired alkaline silver-ionic water only can be taken out from the cathode compartment

of the second electrolytic cell chamber.

Fig. 1 illustrates an example of the apparatus for producing silver-ionic water according to the first embodiment of the present invention.

The apparatus of Fig. 1 chiefly comprises a first electrolytic cell chamber 11 and a second electrolytic cell chamber 21. The first electrolytic cell chamber 11 is enclosed by a bottom plate 12 comprising a non-conductive material, a cathode plate 13 surrounding the outer periphery and made of stainless steel or the like, and a cover plate 14 made of a non-conductive material. Through the cover plate 14, an anode rod 15 is inserted in the manner that the lower end portion thereof may be inward extended. Also, the anode rod 15 is provided with silver or silver alloy 16.

To the bottom plate 12, a first water-feeding pipe 31 is connected via a flowmeter 32 provided on the way of the pipe so that water may be introduced into the first electrolytic cell chamber 11 through the pipe 31. To the cover plate 14, connecting pipes 33 and 34 are connected so that the water in the first electrolytic cell chamber 11 can be led out of it. The connecting pipe 33 is provided with a valve 35 and the connecting pipe 34 is provided with a valve 36.

On the other hand, the second electrolytic cell

chamber 21 is enclosed by a bottom plate 22 made of a non-conductive material, a cathode plate 23 surrounding the outer periphery and made of stainless steel or the like, and a cover plate 24 made of a non-conductive material. To the cover plate 24, an anode plate 25 is fixed in the manner that it may be inward extended. In the inside of the second electrolytic cell chamber 21, a cylindrical diaphragm 26 is arranged so as to surround the anode plate 25, which diaphragm 26 partitions an anode compartment 27 and a cathode compartment 28. The diaphragm 26 has properties that it allows ions such as $Ca^{++}$, $Mg^{++}$, $Na^{+}$, $K^{+}$, etc. to pass from the anode compartment 27 to the cathode compartment 28 and allows ions such as $Cl^{-}$, $SO4^{--}$, $HCO_3^{-}$, etc. to pass from the cathode compartment 28 to the anode compartment 27, to prevent reverse flowing of them.

The aforesaid connecting pipes 33 and 34 are connected to the bottom plate 22, of which the connecting pipe 33 is led to the above cathode compartment 28 and the connecting pipe 34 is led to the above anode compartment 27. Lead-out pipes 37 and 38 are connected to the cover plate 24, of which the lead-out pipe 37 is led to the cathode compartment 28 and the lead-out pipe 38 is led to the anode compartment 27. The lead-out pipes 37 and 38 are provided with valves 39 and 40, respectively.

A second water-feeding pipe 41 is connected between

the valve 35 of the connecting pipe 34 and the bottom plate 22 via a flowmeter 42 and a valve 43. A third water-feeding pipe 44 is connected between the valve 36 of of the connecting pipe 34 and the bottom plate 22 via a flowmeter 44 and a valve 46.

To simultaneously obtain the acidic silver-ionic water and the alkaline silver-ionic water by use of this apparatus, valves 35, 36, 39 and 40 are opened, and valves 43 and 46 are closed, so that water is fed from the first water-feeding pipe 31. The water thus fed from the first water-feeding pipe 31 is led into the first electrolytic cell chamber 11, where silver ions are formed. Then, part of the water containing silver ions is led into the anode compartment 27 of the second electrolytic cell chamber 21 through the connecting pipe 34, and taken out from the lead-out pipe 38 in the form of acidic silver-ionic (e.g. $Ag^+$) water formed in the anode compartment 27 and having an electric conductance of 350 to 2000 $\mu\text{U}/cm^3$. At the same time therewith, remaining part of the water containing silver ions is led into the cathode compartment 28 of the second electrolytic cell chamber 21 through the connecting pipe 33, and taken out from the lead-out pipe 37 in the form of alkaline silver-ionic (e.g. $Ag(OH)_2^-$, $Ag_2(OH)^+$) water formed in the cathode compartment 28.

To obtain only the acidic silver-ionic water by use

of the above apparatus, procedures may be taken as follows: Valves 36, 39, 40 and 43 are opened, and valves 35 and 46 are closed. Under such a state, water is fed under pressure from the first water-feeding pipe 31 and the second water-feeding pipe 41, respectively. The water fed from the first water-feeding pipe 31 passes through the first electrolytic cell chamber 11, the connecting pipe 34 and the anode compartment 27 of the second electrolytic cell chamber 21, and flows out from the lead-out pipe 38. Also, the water fed from the second water-feeding pipe 41 passes through the connecting pipe 33 and the cathode compartment 28 of the second electrolytic cell chamber 21, and flows out from the lead-out pipe 37. Here, the flow rate of the water flowing through both the flow paths is controlled as necessary while watching the flowmeters 32 and 42. Under such a state, a direct-current voltage is applied between the anode rod 15 and cathode plate 13 of the first electrolytic cell chamber 11, and also a direct-current voltage is applied between the anode plate 25 and cathode plate 23 of the second electrolytic cell chamber 21. Taking these procedures, the water fed from the first water-feeding pipe 31 is led into the first electrolytic cell chamber 11, where silver ions are formed, led into the anode compartment 27 of the second electrolytic cell chamber 21 through the connecting

pipe 34, and taken out from the lead-out pipe 38 in the form of acidic silver-ionic water such as $Ag^+$ ionic water formed in the anode compartment 27 and having an electric conductance of 350 to 2000 u $/cm^3$.

To obtain only the alkaline silver-ionic water by use of the above apparatus, valves 35, 39, 40 and 46 are open, and valves 36 and 43 are closed. Water is fed from the first water-feeding pipe 31 and the third water-feeding pipe 44, respectively. Taking these procedures, the water fed from the first water-feeding pipe 31 is led to the first electrolytic cell chamber 11, where silver ions are formed, led into the cathode compartment 28 of the second electrolytic cell chamber 21 through the connecting pipe 33, and taken out from the lead-out pipe 39 in the form of alkaline silver-ionic water such as $Ag(OH)_2^-$ or $Ag_2(OH)^+$ water formed in the cathode compartment 28.

In this apparatus, acidic or alkaline silver-ionic water having a desired silver ion concentration and pH can be obtained by controlling the flow rate of the water flowing though the respective flow paths, the voltage to be applied to the anode rod 15 and cathode plate 13 of the first electrolytic cell chamber 11 and the voltage to be applied to the anode plate 25 and cathode plate 23 of the second electrolytic cell chamber 21.

In this instance, the controlling of the silver ion concentration can be varied chiefly depending on the voltage applied to the anode rod 15 and the cathode plate 13 of the first electrolytic cell chamber 11. Speaking more precisely, the silver ion concentration can be controlled according to the product of the applied voltage in the first electrolytic cell chamber 11 and the electric current flowing in water at that time; in other words, the electric power consumption. Since the electric current flowing in water can be determined according to the applied voltage, the electric conductance of water and the flow rate of water, it anyway follows that the silver ion concentration can be controlled according to the applied voltage, the electric conductance of water and the flow rate of water. Accordingly, it follows that the higher the applied voltage, the electric conductance of water and the flow rate of water are, the higher the silver ion concentration is made.

Experiments were actually carried out by use of the above apparatus to reveal the relationship between the electric power consumption in the first electrolytic cell chamber 11 and the silver ion concentration of the water taken out from the second electrolytic cell chamber 21, which is as shown in Table 1. It is seen from Table 1 that the higher the electric power consumption in the

first electrolytic cell chamber is, the higher the silver ion concentration is.

Table 1

| Electric power consumption (W) | Water flow rate (lit/min) | Silver ion concentration (ppm) |
|---|---|---|
| 1 | 75 | 0.03 |
| 1 | 4.5 | 0.5 |
| 6.8 | 4.5 | 1 |
| 12.6 | 4.5 | 1.5 |
| 15.5 | 4.5 | 2 |
| 20.5 | 4.5 | 2.5 |
| 22.5 | 4.5 | 3 |
| 24.5 | 4.5 | 3.5 |
| 26 | 4.5 | 4 |
| 28 | 4.5 | 4.5 |
| 30 | 4.5 | 5 |
| 30 | 2.25 | 10 |

In the apparatus of the present invention, the pH can be chiefly controlled by the electric power consumption in the second electrolytic cell chamber 21. This electric power consumption can be determined by the voltage applied to the anode plate 25 and the cathode

plate 23, and the electric current flowing in water. The electric current flowing in water can be varied depending on the flow rate of water and the electric conductance of water, when the applied voltage is kept constant. Now, the voltage applied to the second electrolytic cell chamber 21 was made constant at 100 V, with varied flow rate of the water flowing in the anode compartment 27 and the cathode compartment 28, and the pH of the acidic silver-ionic water taken out from the anode compartment 27 and the pH of the alkaline silver-ionic water taken out from the cathode compartment 28 were respectively measured. Results obtained are shown in Table 1a (the acidic silver-ionic water) and Table 1b (the alkaline silver-ionic water). Table 1a and Table 1b tell that the smaller the flow rate is, the more the electric current is, and, as a result, the higher the electric power consumption is. It is seen from Table 1a that the pH decreases with increase in the electric power consumption, in respect of the acidic silver-ionic water taken out from the anode compartment 27. It is also seen from Table 1b that the pH increases with increase in the electric power consumption, in respect of the alkaline silver-ionic water taken out from the cathode compartment 28.

Table 1a

(Acidic silver-ionic water)

| Flow rate (lit/min) | Voltage (V) | Current (A) | pH value |
|---|---|---|---|
| 10 | 100 | 4 | 5.13 |
| 9 | 100 | 5 | 4.25 |
| 2 | 100 | 12 | 3.29 |

Table 1b

(Alkaline silver-ionic water)

| Flow rate (lit/min) | Voltage (V) | Current (A) | pH value |
|---|---|---|---|
| 10 | 100 | 4 | 7.90 |
| 9 | 100 | 5 | 9.09 |
| 4.5 | 100 | 12 | 10.5 |

A 1st use of the silver-ionic water of the present invention is concerned with a bacteriostatic water which can be used in the field of, for example, sanitary administration in processing, distribution and selling of food, soil conditioning in agriculture, environmental sanitation in medical care, etc. Particularly, it is concerned with bacteriostatic water, which contains silver ions in given concentration under particular pH

conditions, so that it can be less dangerous to human bodies and can have an excellent bacteriostatic effect against bacteria mixedly present together with proteins and also an excellent bacteriostatic effect against thick spore membrane type microorganisms.

The change in economic structure, called the distribution revolution, has brought about a food mass-processing system, along with which food additives such as food preservatives and food colorants have acceleratedly trended toward wider use. Also, environmental pollutions as in kitchens may invite a danger of sitotoxism or tend to widely cause wretched sitotoxism.

Agents used for preventing these are governed in accordance with the safety standard for chemicals and food additives. However, according to detailed surveys, the standard is merely a standard of the degree of dangers to humans presently being alive, and any disorders in a fetus that may affect next generations or any influences to genetic factors have not been made clear.

A great number of mutable sources, carcinogenic substances and so forth that may have certain concerns with the hereditary disorders has been progressively analysed also from commercially available chemically synthesized agents. Table 2 reports an investigation result obtained by carrying out tests for repair to DNA

and mutation induction of Escherichia coli, in respect of various substances contained in the commercially available products. In the table, the symbol "+" represents the strength of the action of mutation induction, and "-" shows that there was no action of mutation induction.

This investigation has revealed that the synthetic chemical substances such as arsenic, cadmium, chromium, mercury, molibdenum, manganese, selenium, tellurium and thallium are the mutable sources. Most of the harmful effect is caused by the mechanism such that toxic ions are combined with functional groups that can effect a metabolic reaction important to a living body, and substituted with essential trace ions. Also, the harmfulness may depends on not only the properties of these toxic ions but also the rate of absorption, distribution, deposition, excretion or the like.

In human bodies, there are four elementary groups of the essential, significant, incidental and toxic elements. The essential elements include 18 elements consisting of H, Na, K, Mg, Ca, Mo, Mn, Fe, Co, Cu, Zn, C, N, P, O, S, Cl and I. The significant elements include 8 elements consisting of V, Cr, Ni, Si, Sn, Se, F and Br. Further, 20 to 30 elements are distributed as the incidental elements. Of these, those which may cause physiological and behaviological modulations at the concentration higher

than a certain level are called toxic elements. However, there remain many fields in which the physiological role depending on the concentration has not been made clear.

Now, in the natural environment, the phenomenon in which a cell membrane itself is suddenly inactivated is such that once a peripheral ion type substance which is a biosubstance capable of readily oxidized with Fe ions, or a -OH ion, is generated in contiguity with the membrane, hydrogen is withdrawn from a polyvalent unsaturated aliphatic acid to give a radical of a spindle saturated aliphatic acid, which radical is combined with biradical oxygen to give an aliphatic peroxy radical, whereupon the peroxidation decomposition takes place one after another and the membrane is finally inactivated.

In order to artificially cause this phenomenon, a catalyst is required by which these chemical reactions are allowed to take place. As a result of the surveys made by the present inventors, no metal having this function has been found other than silver. In the oxidation reaction of ethylene, the silver has a catalytic action to form 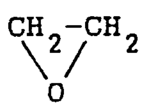 as a partially oxidized product, which action is a selective action that can not be substitutionally achieved by other metals, and can be very specific also in respect of selective absorption functions for gases as shown in

Table 4.

Utilizing this catalytic function, the silver may be acted on an aliphatic acid of a cell membrane to cause the phenomenon that an alcohol is formed as $C_2H_5OH$, so that a change in the membrane structure may occur to inactivate the function, the molecules of water in the first layer adhered to a protein can be eliminated, and the tertiary structure can be destroyed, whereupon the enzymatic activity can also be inactivated.

However, when the silver is used, it is necessary to examine in detail the adverse influence that the silver element may give to living bodies. Thus, examined was how the typical metallic ions behave against complex-forming atoms in an aqueous solution, to obtain the results grouped as shown in Table 3. In particular, silver ions belong to the soft metallic ion, and have characteristic properties that they form only a weak bond to the hard ligand $H_2O$ to have no solubility. This means that, in general, the silver forms a weak bond to bioproteins, showing a character that it can be hardly incorporated into living bodies.

For example, an experiment for measurment of concentration in the bodies of scallops (Patinopecten yessoensis) lived in sea water containing silver and mercury in concentration of 0.04 ppb each, revealed that

the silver concentration in the bodies was 5.3 ± 0.8 ppm (wet weight) and the mercury concentration in the bodies was 48.9 ± 5.9 ppm (wet weight). This proves that silver is an element having a quite different nature as being hardly incorporated into living bodies.

From a viewpoint of clinical medicine, a soluble silver salt has a factor adverse to human bodies, but, reportedly, an insoluble silver salt has no remarkable toxicity. A report, "Hifu-Rinsho (Dermatoclinic)", 20(12), 1013-1018, 1978, also reports that no particular physiological disorder was seen in a medical clinical examination on a patient suffered from chromatosis argrya caused by a long term administration of 1,200 to 1,300 grains of "Jintan" (trademark; a multiple-purpose oral sanitary drug available from Morishita-Jintan K.K., comprising a grain of about 2 mm in diameter, containing various herbs or crude drugs, and provided on its surface with a silver coating of about 3,000 angstroms thick, formed, for example, by vacuum deposition of silver to have a coating amount of about 0.1 mg/grain), calculated as 120 to 130 mg of silver, a day.

Table 2

| | | | | | |
|---|---|---|---|---|---|
| AgCl | – | K3[Fe(CN)$_6$] | – | Pb(CH$_3$COO)2 | – |
| $AlCl_3$ | – | $K_4[Fe(CN)_6]$ | – | $PbCl_2$ | – |
| $AsCl_3$ | + | HgCl | – | $PdCl_2$ | – |
| $NaAsO_2$ | + | $HgCl_2$ | – | $RhCl_2$ | – |
| $Na_2HAsO_4$ | – | $CH_3HgCl$ | + | $RuCl_3$ | – |
| $BaCl_2$ | – | $CH_3COOHgC_6H_5$ | + | $K_2SeO_3$ | + |
| $BeCl_2$ | – | $LaCl_3$ | – | $K_2SeO_4$ | + |
| $BiCl_3$ | – | LiCl | – | $SbCl_3$ | – |
| $CdCl_2$ | + | $MgCl_2$ | – | $SbCl_5$ | – |
| $Cd(NO_3)_2$ | – | $MnCl_2$ | + | $SnCl_2$ | – |
| $CoCl_3$ | – | $Mn(NO_3)_2$ | + | $SnCl_4$ | – |
| $CoCl_2$ | – | $MnSO_4$ | + | $Na_2SnO_3$ | – |
| CuCl | – | $Mn(CH_3COO)_2$ | + | $TbCl_3$ | – |
| $CuCl_2$ | – | $KMnO_4$ | – | $TeCl_4$ | – |
| $CrCl_3$ | – | $MoCl_5$ | – | $K_2TeO_4$ | + |
| CsCl | + | $K_2MoO_4$ | + | $Na_2H_4TeO_6$ | + |
| $K_2CrO_4$ | ++ | $(NH_4)_6Mo_7O_{24}$ | ++ | $ThCl_4$ | – |
| $K_2Cr_2O_7$ | +++ | $NbCl_5$ | – | TlCl | + |
| $FeCl_2$ | – | $NiCl_2$ | – | $ZnCl_2$ | – |
| $FeCl_3$ | – | $PbCl_2$ | – | | |

Table 3

| | |
|---|---|
| **Classification of cation species:** | |
| (a): | $H^+$, $Li^+$, $Na^+$, $K^+$, $Mg^{2+}$, $Ca^{2+}$, $Mn^{2+}$, $Cr^{3+}$, $Fe^{3+}$, $Co^{3+}$ |
| Intermediate: | $Zn^{2+}$, $Cu^{2+}$, $Ni^{2+}$, $Fe^{2+}$, $Co^{2+}$, $Sn^{2+}$, $Pb^{2+}$ |
| (b): | $Cu^+$, $Ag^+$, $Au^+$, $Tl^+$, $Pb^{2+}$, $Pt^{2+}$, $Cd^{2+}$ |
| **Classification of anion species:** | |
| (a): | $H_2O$, $OH^-$, ROH, $OR^-$, $R_2O$, $NH_3$, $-NCS^-$, $Cl^-$, $PO_4^{3-}$, $SO_4^{2-}$, $F^-$, $NO_3^-$, $CO_3^{2-}$ |
| Intermediate: | 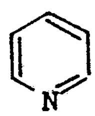 , $RNH_2$, $N_2$, $N_3^-$, $NO_2^-$, $Br^-$ |
| (b): | RSH, $RS^-$, $R_2S$, $R_3P$, $R_3As$, CO, $-CN^-$, $-SCN^-$, $S_2O_3^{2-}$, $H^-$, $I^-$ |

(R = alkyl group)

Table 4

| Gas or metal | Ru | Rh | Pd | Ag | Re | Os | Ir | Pt | Au |
|---|---|---|---|---|---|---|---|---|---|
| $H_2$ | ~118 | 117 | 117 | – | + | + | 109 | 109 | – |
| $O_2$ | + | 503 | 294 | + | 732 | + | + | 294 | – |
| $N_2$ | | – | – | – | >210 | (+) | 243 | – | – |
| CO | + | 193 | 180 | – | + | + | + | 201 | 36 |
| $CO_2$ | – | – | – | – | (+) | | (–) | – | (–) |
| $CH_4$, $C_2H_6$ | + | + | + | – | + | (+) | + | + | |
| $C_2H_4$ | + | + | + | 36 | + | (+) | + | + | 87 |

Adsorption temperature: About room temperature

+ : Chemical adsorption, possible

– : Chemical adsorption, difficult

Thus, it has been ascertained that silver ions are very little dangerous for living bodies, and can be used as substances having the bacteriostatic action as aimed in the present invention.

There has been known a large number of examples showing bectericidal effect possessed by silver-ionic water, which is known to have been utilized for the sterilization of water in water tanks or water bottles (or canteens).

However, in the case the water contains proteins or

amino acids, or bacteria adhere to the proteins, there has been involved a serious disadvantage that the silver ions are weakly combined with the proteins or amino acids to discharge electrons so that the bacteriostatic or bactericidal effect may become little obtainable. This is because even if silver ions try to enter the inside of cells of bacteria to achieve the bactericidal action, the substances such as proteins and amino acids already present in the water are combined with the silver ions to discharge ions, so that no effect is obtainable against the bacteria.

Accordingly, another object of the present invention is to provide a bacteriostatic water that may not adversely affect any life system such as human bodies, and that the activity of silver ions against bacteria may not be lowered even if the bacteria are present together with proteins.

The bacteriostatic water of the present invention is characterized by comprising silver-ionic water having the pH of 5 or less or the pH of 8 or more and silver ion concentration of 200 to 2,000 ppb.

The present invention also provides a method for bacteriostatic treatment, comprising allowing silver-ionic water having the pH of 5 or less or the pH of 8 or more and silver ion concentration of 200 to 2,000 ppb, to float

in air in the form of misty waterdrops.

Thus, under the condition of pH 5 or less or pH 8 or more, it is possible to prevent silver ions being adsorbed on proteins present mixedly, thereby preventing a lowering of the activities of ions even with the co-presence of proteins. The fact that the silver ions may not be adsorbed on the proteins may also mean that the silver ions can freely permeate membranes of bacteria. As a result, the bacteriostatic action can be enhanced. Moreover, under the condition of the silver ion concentration of 200 to 2,000 ppb, cells may not be killed completely and can be inactivated retaining the active functions thereof. The silver ion concentration less than 200 ppb may result in no sufficient bacteriostatic effect obtained, and the silver ion concentration more than 2,000 ppb may result in a overly strong bacteriostatic action to give a bactericidal action. It is detrimental to the life system, particularly to human bodies, to kill even the bacteria that can be useful from a medical viewpoint.

According to a preferred embodiment of the 1st use of the present invention, the bacteriostatic water has an electrical conductance of 350 to 2,000 $\mu U/cm^3$ in the case of the condition of pH 5 or less. Thus, by adjusting the electrochemical potential to the above range, it is made possible to retain the ionic activities of silver ions

even against the bacteria present together with proteins or the bacteria to which proteins have been attached as finely particulate water droplets, thereby maintaining the bacteriostatic power against target bacteria.

The bacteriostatic water of the present invention can be used in wide fields such as a medical field and a field of processing, distribution and selling of food. For example, in the medical field, for the purpose of keeping sanitary the bodies of medical workers or patients or keeping sanitary the medical utensils, clothes, sheets, etc., the bacteriostatic water can be utilized by dipping them in the bacteriostatic water or spraying it in the form of mist. Also, in the field of food, this bacteriostatic water can be used as water to be added to food materials, can be used by spraying the bacteriostatic water in the form of mist on packages of food, or can be used as water for washing food production machines, transporting appliances, etc. In particular, in the case the bacteriostatic water is allowed to float in air in the form of mist, the effect against bacteria present inside of the surface layer of meat, fish, etc. can be heightened by increasing the electrochemical potential, whereby the bacteriostatic water can permeate the cells at the surface layer of the food to have the silver ion effect on the bacteria.

[Examples of the 1st use]

The present inventors, who have ascertained that the presence of $[H^+]$ protons greatly participates in the combination of trace elements with proteins without regard to the kind of the trace elements and that the combination is effected in a similar manner in a given range of concentration, have carried out the following experiments to observe the combination activity in living bodies.

Serum albumin is known to combine with acetyl-L-tryptophan in the ratio of 1 : 1. Now, the influence of salt concentration and pH, that may give to the combination of serum albumin with acetyl-L-tryptophan, was examined to obtain the results as shown in Fig. 2. In Fig. 2, the curve with "o-o" shows a result obtained in an aqueous solution of 0.01M KCl; the curve with "●-●" a result obtained in an aqueous solution of 0.1M KCl; and the dotted line an electrostatic repulsion force with increase in negative charge, obtained from an apparent combination constant (K')

It is seen from the above results that the combination decreases at pH 5 or less and pH 8 or more whatever the concentration is. This is presumably because, in view of the change in the electrophoresis of serum albumin and the pH titration, carboxyl groups present in the inside of proteins cause structural change

of the proteins at about pH 5 when the pH is neutral, and, on the other hand, lysine residual groups or arginine residual groups in the proteins begin to be released therefrom at about pH 8 to lower the combination ability.

In general, the combination of amino acids and metallic ions is known to be a reaction in which protons are released from amino acids having been turned protonic, with which metallic ions are substituted, and the degree of combination with the metallic ion is considered to be made greater with an increase in the pH. This is considered to be for the following reasons: As a result of release of the protons from dissociative side chains of proteins, the number of negative charge in proteins increases to make it easier to be combined with metallic ions, and, at a higher pH value, competing with metallic ions, the amount of protons combining with proteins becomes smaller to make it easier to be combined with the metallic ions. This theory is in conformity with the experiment on serum albumin.

Based on these experiments and theoretical verification, the present inventors have considered that the condition of pH 5 or less or pH 8 or more should be effective for maintaining the silver ion activities. Now, with use of the apparatus as shown in Fig. 1, silver-ionic water was produced with varied pH and silver ion

concentration, to evaluate the bacteriostatic action thereof.

Using the above apparatus, acidic silver-ionic water each having the pH of 3.5 and a silver ion concentration of 800 ppb, 1,200 ppb, 1,600 ppb and 2,000 ppb was prepared. Thereafter, a platinum loop each of Escherichia coli and Staphylococcus was inoculated into each solution, followed by addition of a 0.1 % broth, and the movements (viable cell counts) of the bacteria were observed with time lapse while culturing them at 35°C. As a result, the viable cell counts on Escherichia coli were as shown in Table 5.

As to Staphylococcus, the state of growth thereof was observed based on the turbidity of the solutions to obtain the results as shown in Table 6. Thus, the acidic silver-ionic water was found to have a remarkable bacteriostatic action.

The Escherichia coli and Staphylococcus used in the above were further cultured to repeat experiments similar to the above several times, but there appeared no bacteria resistant to silver ions, showing the same bacteriostatic action as in the first instance. Thus, there is a feature noticeable from a medical viewpoint, that the appearance of resistant bacteria, which has been very usual in using conventional medicaments, is not seen at all in using this

silver ions.

Table 5

| Contact time | Silver ion concentration | | | | |
|---|---|---|---|---|---|
| | Control 0 | 800 ppb | 1200 ppb | 1600 ppb | 2000 ppb |
| 10 min | 2000 | 160 | 290 | 60 | 180 |
| 2 hrs | 3500 | 70 | 70 | 30 | 30 |
| 4 hrs | 20000 | 30 | 20 | 0 | 0 |
| 6 hrs | 55000 | 20 | 10 | 0 | 0 |
| 24 hrs | 19000000 | 30 | 0 | 0 | 0 |
| 48 hrs | 80000000 | 500000 | 500000 | 500000 | 0 |

Table.6

Tests on Staphylococcus suspension

| Contact time | Control | 800 | 1200 | 1600 | 2000 ppb |
|---|---|---|---|---|---|
| 24 hrs | + | – | – | – | – |
| 48 hrs | + | + | – | – | – |
| 72 hrs | + | + | – | – | – |

Next, prepared was silver-ionic water each having a silver ion concentration of 1 ppm, 0.5 ppm and 0.2 ppm, the pH of which was varied respectively to be 10, 9, 8, 7, 6, 5, 4 and 3. On the other hand, Staphylococcus and

Escherichia coli were subcultured for 3 days with use of an ordinary broth medium. Also, general various bacteria were cultured from white coffee. The above Staphylococcus, Escherichia coli and general various bacteria were collected by the number of $10^5$ for each, and brought into contact with each of the above silver-ionic water for 5 minutes to measure the viable cell counts. Results obtained were as shown in Table 7. As will be seen from Table 7, the bacteriostatic action tends to become remarkable at the pH of 5 or less or the pH of 8 or more. Fig. 3 is a graphic illustration of the above results on Staphylococcus, Fig. 4 is a graphic illustration of the above results on Escherichia coli, and Fig. 5 is a graphic illustration of the above results on general various bacteria.

Table 7

| Ag ion concentration: | Staphylococcus | | | Escherichia coli | | | General various bacteria | | |
|---|---|---|---|---|---|---|---|---|---|
| | 0.2 ppm | 0.5 ppm | 1 ppm | 0.2 ppm | 0.5 ppm | 1 ppm | 0.2 ppm | 0.5 ppm | 1 ppm |
| pH 10 | 0 | 0 | 0 | 0 | 0 | 0 | 0 | 0 | 0 |
| pH 9 | 10 | 0 | 0 | 70 | 0 | 0 | $8.9x10^3$ | 0 | 0 |
| pH 8 | $1.1x10^4$ | 43 | 1 | $9.1x10^3$ | 2 | 1 | $2.1x10^4$ | $10x10^3$ | $2.0x10^2$ |
| pH 7 | $5.3x10^4$ | $3.7x10^3$ | $3.6x10^2$ | $3.6x10^4$ | $9.4x10^3$ | $5.6x10^2$ | $8.2x10^4$ | $6.4x10^3$ | $2.5x10^2$ |
| pH 6 | $9.8x10^3$ | $1.1x10^3$ | $2.1x10^2$ | $2.1x10^4$ | $6.2x10^3$ | $3.1x10^2$ | $6.1x10^4$ | $5.1x10^3$ | $1.9x10^2$ |
| pH 5 | $5.5x10^3$ | $8.2x10^3$ | 27 | $8.7x10^3$ | $7.2x10^2$ | 50 | $9.1x10^3$ | $2.6x10^3$ | $1.1x10^2$ |
| pH 4 | $4.2x10^3$ | 77 | 3 | $3.6x10^3$ | 12 | 0 | $4.5x10^3$ | 610 | 0 |
| pH 3 | 10 | 0 | 0 | 2 | 0 | 0 | 87 | 0 | 0 |

The above experiments verified the conformity with the experiments for serum albumin and acetyl-L-tryptophan mentioned above. Specifically, the combination of metallic ions with proteins under the condition of pH 5 or less or pH 8 or more may occur only with very low probability, whereby it becomes possible for silver ions in concentration of 200 to 2,000 ppb to be in contact with bacteria as they are. And, silver ions can readily flow into the inside of cells through the portion where the electric resistance of the hydrophilic part of cell membranes of bacteria is as low as $10^2$ to $10^5\ \Omega/cm^2$, to cause a radical reaction in the polyvalent unsaturated aliphatic acid at the hydrophobic part by virtue of the catalytic function of silver. More specifically, the cell membranes of bacteria, which are cis-type sequence of ethylene, may accelerate the combination of hydroxyl groups to alkyl groups to be formed into alcohols, thereby making the molecular activity greater, so that hydrogen is withdrawn from the polyvalent unsaturated aliphatic acid, taking place the radical reaction, to combine with oxygen which is biradical, and finally peroxidation decomposition takes place. As a result thereof, the cell membranes of bacteria lose their functions, so that water flows thereinto and the bioactivities thereof are stopped. On the other hand, substances constituting DNA and the

nuclear membrane may not be affected by the catalytic action of silver, may be retained in a sound form and can be activated to begin to undergo cell division when cultured again. Thus, the so-called bacteriostatic effect can be achieved.

Accordingly, this silver-ionic water water can be used in the preservation of food, sterilization of tableware or kitchens, washing of food, sterilization of the inside of refrigerators and so forth. For example, this silver-ionic water may be formed into mist with use of a high frequency oscilator to be allowed to float in the space in the inside of refrigerators or the space in display racks in supermarkets, whereby there can be expected the effect of preventing putrifaction of food or sitotoxism.

The aforementioned electrolytic cell of Katadyn system, in which electrolysis is carried out by applying a direct-current voltage to an equipment comprising an anode provided with silver and a cathode disposed with an electroconductive electrode, can produce ionic water in a water tank, containing silver ions present together with other ions such as potassium ions, sodium ions, magnesium ions, chloride ions, sulfate ions and bicarbonate ions. This ionic water was added to bacteria in an aqueous solution in which proteins have been dissolved and to

bacteria adhered to proteins, but, because of its neutral environment, the metallic ions and proteins vigorously combined and the silver ions, discharging ions, could not enter the cells of bacteria. As a result, the effect of silver ions was lost without achieving any object.

Also, in the aforesaid apparatus disclosed in Japanese Unexamined Patent Publication No. 97088/1985, a great electric power was required to achieve the desired pH of pH 5 or less and pH 8 or more, so that silver particles precipitated to form colloids of silver particles, and the silver-ionic water thus obtained was of no use.

Thus, the bacteriostatic water of the present invention can be obtained for the first time by use of the apparatus as shown in Fig. 1, and can not be obtained by the conventional apparatus for producing silver-ionic water.

As described above, in the present invention, the pH is made to be 5 or less or 8 or more and the silver ion concentration is made to be 200 to 2,000 ppb. Accordingly, it is possible to prevent silver ions being adsorbed on proteins, and it is also possible to achieve remarkable bacteriostatic effect with relatively lower ion concentration even when proteins are present together with bacteria. Also, the bacteriostatic water according to the

present invention may not spoil DNA or cell membranes, may not completely kill the functions of cells, and may have a bacteriostatic action that can inhibit the growth of bacteria. This can again activate the bacteria harmless to human bodies, and is very little dangerous. Accordingly, this can be used without anxiety in the medical field or the field of food. Still also, as the electrical conductance is made to be 350 to 2,000 µmU/cm$^3$, the electrochemical potential can be increased, so that it is made possible for silver ions to enter the bodies of bacteria present in food in such a form of thick membrane spores. Moreover, this bacteriostatic water can be allowed to float in the form of mist, whereby it is made possible to have the bacteriostatic action also against bacteria present in the vicinity of the surface layer of food to achieve more sanitary preservation.

A 2nd use of the silver-ionic water of the present invention is concerned with a plant growth promotive acidic water for cultivation of agricultural products (hereinafter "growth promotive acidic water", comprising silver-ionic water that can have effect such as soil-conditioning, controlling of pathogenic bacteria and growing of symbiotic bacteria, promotion of the growth and maturation of fruit, and so forth.

In cultivation of agricultural products, it is well known that various types of soil-conditioning agents, agricultural chemicals and growth promotive agents have been used.

In the greenhouse soil, evaporation of water may be accelerated because of the high temperature and the water moves from lower part to upper part according to an capillary action, along with which salts dissolved in the water may move and gather in the vicinity of the ground surface and may crystalize at the ground surface by the evaporation of water, so that the salt concentration may be extraordinarily increased.

On the other hand, the soil tends to turn acidic by the chemical reaction of chemical fertilizers repetitively applied in the soil. There may occur the oxidation caused by the soil and fertilizers immediately after the application of fertilizers, the oxidation caused subsequently by the nitrous acid formation of $NH_4$-N, and so forth. In a greenhouse, no phenomenon of washing-away and neutralization can be expected, and therefore, the soil may have complicated soil properties such that crystals of salts and colloids which turned acidic are present together in the form of conglomerates, so that it may often occur that the roots of crops suffer from a disorder by salt concentration or that the activities of

microorganisms having a symbiotic relation to the roots may be weakened.

The greenhouse may also give an environment of high temperature and high humidity, where fungi may propagate themselves to inhibit the respiration of plants, and, in the soil, Fusaria may propagate themselves to cause root rot, not only giving great damage to crops but also making it impossible to repeat cultivation of the same crops on the same ground.

To solve these problems in the greenhouse soil, it has been conventionally attempted to wash away the salts by sprinkling of a large quantity of water, but the salts temporarily having been forced down to a lower portion together with the sprinkled water may return to the ground surface anyway. Accordingly, it has been impossible to break out this antagonism.

There would be no other way than to turn and take over the greenhouse soil at a great cost to carry out soil replacement. However, the soil, even if replaced with new one with much effort, may return to the previous state after lapse of a certain period, without giving any fundamental resolution.

Also, a variety of chemical pesticides is sprayed against pathogenic bacteria in an acidic soil. However, not only no effect may not be seen against those which can

be resistant to agricultural chemicals as mold fungi can, those which can survive by changing their own life system in conformity with surroundings as slime mold fungi can, and thosemicroorganisms capable of hybanating by transforming themselves into thick membrane spores, but also resistant bacteria may be sometimes developed. Thus, there can be a great damage at the time of harvesting. Moreover, the frequent use of agricultural chemicals may always provide a potential danger of serious damage on the health of humans.

Next, the acidic soil in cultivated fields which is seen in the bare ground cultivation or in fruit gardens, has an environment rather different from that of the greenhouse cultivation.

Once the soil is sprinkled by a large quantity of rainwater, sodium and potassium weakly combined with soil colloids through positive arms are so readily compatible with water that they may be immediately released from the soil colloids to take such a form that they are surrounded by negatively charged arms of the molecules of water, mutually repulse the negative charge in the soil colloids, and be readily scattered and washed away.

Calcium and magnesium are so strongly combined with the soil colloids that they may not be released by a smaller quantity of water and may not be readily

scattered. However, at last they may be washed away with lapse of time as non-active metals.

In this manner, in the soil as a whole, $H^+$ ions are replaced with metallic ions, so that the soil may turn to an acidic soil having high hydrogen ion concentration and not a soil having been well balanced. Moreover, the oxygen content in the ground is so low that the soil may be sometimes occupied with pathogenic bacteria having dominated symbiotic bacteria. Thus, the growth of plants may be extremely inhibited.

The greenhouse nutrient solution cultivation is a method of crop cultivation separated from the soil, and can be roughly classified into the mist culture, the hydroponics (or soilless culture) and the solid medium culture, which aim at factory production of agricultural products. However, in stone culture or sand culture, it is difficult to treat remaining roots, which remaining roots may become sources of the contamination by pathogenic bacteria, so that diseases may be very frequently caused and, once they happened, may rapidly spread over the whole crops. Moreover, since the nutrient solution for the cultivation has no oxygen-dissolving ability the soil otherwise has, there may be brought about a state of lack in oxygen, causing the plant growth inhibition.

To solve these problems, chemical agent administration, soil replacement or the like has been carried out. However, no effective result has been obtained.

Conventionally, it has been attempted to use silver-ionic water as health drink or a sterilizer for swimming pools. However, any of these only aim at the bactericidal effect of silver ions, and there has been no attempt at all to effectively use silver-ionic water in the field of agriculture.

Accordingly, a further object of the present invention is to solve such complicated problems in the soil as those originating from excessive salts or acidic soil in the cultivation of plants such as agricultural products.

A still further object of the present invention is to provide an epock-making growth promotive acidic water that can exterminate pathogenic bacteria in agricultural products or the like without care of adverse effect on human bodies that may be caused by continuous application of agricultural chemicals, can produce an environment ready to grow symbiotic bacteria, and also can have an action to supply sufficient amount of dissolved oxygen necessary for the growth of plants and also to promote the growth and maturation of the fruit part of plants such as

agricultural products.

The growth promotive acidic water of the present invention is characterized by comprising silver-ionic water having the pH of 5 or less, silver ion concentration of 30 to 2,000 ppb, electrical conductance of 350 to 2,000 $\mu\mho/cm^3$ and a dissolved oxygen amount of 12 to 30 ppm.

The growth promotive acidic water of the present invention may be sprayed and permeated into the greenhouse soil, whereby the salts gathered on the surface of the greenhouse soil are dissolved and provided with increased electrochemical potential, so that the hydrogen ions adhered to soil colloids are forced to be replaced by metallic ions in a short time, and the salts are made to be in a free state. Thereafter, sprinkling of water in a large quantity may be carried out to wash away excessive salts toward a lower portion together with water, whereby well-balanced normal solid colloids can be formed.

The growth promotive acidic water of the present invention may also be sprayed and permeated into the bare ground, whereby the inorganic components such as metals contained in the soil can be activated as being provided with increased electrochemical potential, and substituted on the hydrogen ions adhered to soil colloids, so that there can be obtained an effect of forming well-balanced soil colloids abundantly containing inorganic components

necessary for the growth of agricultural products. In this instance, it is impossible that the acidity is increased by the buffering action in the soil.

In the soil cultivation, fertilization is often effected by using organic fertilizers together, and a large number of microorganisms, which may decompose organic substances in the soil, is present in the form of pathogenic bacteria, thick membrane spores or oospores. This may often result in such a state that even a strong agricultural chemicals may have no effect.

The growth promotive acidic water of the present invention can be sprayed and permeated into the soil without any complex formation between proteins and silver ions contained in the soil, can come into contact with pathogenic bacteria and other microorganisms as it is, can flow into the inside of cells through their cell membranes according to the inclination of the electrochemical potential, and can cause a phenomenon in which the cell membranes are made alcoholic by the catalytic action the silver ions have, whereby it is made possible to inactivate the function thereof and remarkably decrease the pathogenic bacteria and other microorganisms.

As a matter of course, the plant symbiotic useful bacteria may be also remarkably decreased, but, once thereafter the roots of plants begin to vigorously grow

and begin to secrete organic substances, only the symbiotic useful bacteria may greatly propagate themselves to produce a sound symbiotic environment for plants. Here is made the most of the time difference before the harmful microorganisms again propagate themselves destroying the stable symbiotic relationship. In the case of the nutrient solution cultivation, the growth promotive acidic water may also be mixed into the nutrient solution, whereby the action to the organic fertilizers or the cell membranes of bacteria or fungi adhered to proteins in the roots, and also the effect of inactivating them, can be achieved in the entirely same fashion.

The growth promotive acidic water of the present invention may also be sprayed on foliage of plants, to obtain the equally remarkable bacteriostatic effect against pathogenic bacteria on the foliage.

The silver-ionic water of the present invention is harmless to human bodies, and free from such dangerousness as the agricultural chemicals may have. Moreover, it is advantageous in that the bacteriostatic effect achievable by the silver-ionic water have nothing to do with the generation of resistant bacteria.

The growth promotive acidic water of the present invention can facilitate remarkable growth of the root portions of plants, and can have the effect that foliage

can be grown thick and growth and maturation of fruit can be promoted to achieve an increased yield.

A great number of tests has proved that hormones formed in the plant living bodies play important roles on the growth of the whole bodies or respective organs of plants. Typical growth hormones may include auxin, gebberellin, cytokinin, etc. having complicated molecular structure, and ethylene which has simplest structure and in the form of a gas. These participate in the growth mechanism of plants while influencing each other.

Of these, auxin is synthesized by amino acid tryptophan having an indole nucleus and being universally present in a free state in the plant cells or in such a form that it is incorporated into protein, and is identified as the molecular structure of indole-3-acetic acid (IAA). IAA is principal auxin, may be inactivated by various plane tissues, and stored in the form of ethyl indoleacetic acid. This also plays a role as a sort of concentration regulator.

Hitherto, the effect directly caused by auxin were interposed by an intermediate stage of the promotion of the synthesis of ethylene. There was also discovered the fact that ethylene has caused the reaction also in respect of auxin. This proves that ethylene is produced in a large quantity at the portion containing a large quantity

of endogenous auxin, and both of them are closely related.

The action of auxin on the cell extension is considered such that auxin has an action of promoting the secretion of $H^+$ ions in stem or coleoptile tissue, and, as a result, lowers the pH in the cell walls, weaken or soften the combination between molecules in the cell walls, thereby increasing the water absorption potential to promote the growth. Greater part of the volume increase occurring in the course of the formation of vacuoles in the cells is owing to the water absorption to protoplast, but, needles to say, a prerequisite thereof is the softening of the cell walls surrounding the plant cells. The extension growth can be caused by immersing coleoptiles or etiolated stem tissue in an aqueous solution of lower pH (about 3.0). This is the so-called acid growth effect, which resembles the growth promotive action by the auxin.

Another phenomenon to be brought about by the effect the auxin may have is the hypertrophic growth of fruit to be accompanied with the promotion of cell division owing to the cooperative effect with cytokinin. Auxin also perticipates in the differentiation growth of roots.

As mentioned above, auxin is greatly concerned with the synthesis of ethylene. Accordingly, auxin is considered to paticipate in the growth of plants in the

interelation to ethylene. Specifically, stems may not grow in the presence of ethylene, and the hypertrophic growth in the longitudinal direction is caused to make stems thick. This is considered to be related to the change in the arrangement of cellulosic microfibrils in the cell walls, and also to the increase in the cellulase activities.

Additionally speaking, ethylene may enhance the climacteric rise (a great respiration prior to the lowering of respiration before senescence) to give the effect of promoting the maturation of fruit.

To summarise the hormone reaction of auxin mentioned above, the growth of the root portions may first occur by the activation of auxin, and then $H^+$ ions are secreted to the stem and coleoptile portions to lower the pH of cell walls and soften the cell walls, whereby the water absorption potential of the cells can be increased, the tempo of the volume increase of cells can be accelerated, and the growth of stems or the hypertrophic growth of fruit can be promoted.

Also, it can be assumed that, owing to the cooperative effect of ethylene, foliage can be made thick, and moreover the effect of promoting the maturation of fruit can be produced.

In order to artificially causing these phenomena,

auxin may be activated by separating the ethylene of the inactivated ethyl indoleacetate stored in the plant tissue. If this can be done, it follows that the effect of ethylene can be simultaneously participated.

For that purpose, the present inventors have considered that, with use of silver which has a catalytic function for 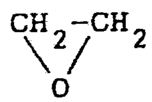 and with provision of an environment of pH 5 or less, there may be utilized the natural mechanism such that active silver ions are allowed to directly act on the cell walls, and the activation of auxin as a foliage growth hormone is achieved, or such that excessive salts are washed away to make the soil colloids normal, the absorption potential of roots is increased by making the osmotic pressure of the soil as lower as possible than the osmotic pressure of the roots (5 to 7 atmospheric pressure), and a large quantity of nutrients is fed to the inside of vacuoles, whereby it would be made possible to make the foliage thick and promote the hypertrophy of fruit. Then, silver-ionic water having the pH of 5 or less, silver ion concentration of 30 to 2,000 ppb, and electrical conductance of 350 to 2,000 $\mu U/cm^3$ was sprayed in the soil. As a result, the electrochemical potential in the soil was increased, the osmotic pressure to the root cells was raised, and the

absorption rate was increased. Ethylene was separated and formed from ethyl indoleacetate in the cells by the selective catalytic function of the silver, and auxin and ethylene were activated, so that root hair was greatly extended, foliage portions became thick vigorously, and the yield of fruit gained a 30 % increase with increased sugar content, successively.

The same effect can be obtained in the bare ground cultivation and the nutrient solution cultivation.

The silver-ionic water constituting the growth promotive acidic water of the present invention is made to have the pH of 5 or less, and silver ion concentration of 30 to 2,000 ppb. The reason why the pH is made to be 5 or less is that the presence of $H^+$ protons greatly participates in the combination between trace elements and proteins, without regard to the kind of the trace elements, and, under the conditions of the pH of more than 5 and less than 8, silver ions tend to form complexes with organic substances such as proteins in the soil or nutrient solution, and be readily absorbed, whereupon there can not be exhibited any bacteriostatic effect or physiological activity effect the silver ions may have.

The reason why the silver ion concentration is made to be 30 to 2,000 ppb is that the concentration of less than 30 ppb may result in no bacteriostatic effect in the

soil or nutrient solution, and the concentration more than 2,000 ppb may result in the inhibition of even the growth of useful bacteria such as symbiotic bacteria.

In a preferred embodiment of this 2nd use of the silver-ionic water of the present invention, the growth promotive acidic water is made to have electric conductance of 350 to 2,000 $\mu\mho/cm^3$. The electric conductance made to be 350 $\mu\mho/cm^3$ or more may increase electrochemical potential, whereby silver ions are made to readily permeate cell membranes of bacteria or the like, and further the hydrogen ions combined with soil colloids can be forced to be substituted with metallic ions. The electric conductance less than 350 $\mu\mho/cm^3$ may result in poorness of the above effect, and the electric conductance more than 2,000 $\mu\mho/cm^3$ may result in difficulty in the production.

According to a more preferred embodiment of this 2nd use of the silver-ionic water of the present invention, the growth promotive acidic water is made to have a dissolved oxygen amount of 12 to 30 ppm. The dissolved oxygen amount increased like this makes it possible to supply agricultural products with oxygen to achieve better growth. Particularly in the case of the nutrient solution cultivation, oxygen is required to be possitively supplied. However, making the dissolved oxygen amount as

above may make it unnecessary to carry out aeration or the like, so that the production cost such as cost for equipments can be greatly decreased. The dissolved oxygen amount less than 12 ppm may result in poorness of the above effect, and that more than 30 ppm may result in difficulty in the production.

The growth promotive acidic water of the present invention can be produced by the apparatus shown in Fig. 1.

Namely, using the apparatus, water may be passed through the first electrolytic cell chamber with application of voltage, where silver ions are dissolved out, and the water thus treated may be passed through the anode compartment of the second electrolytic cell chamber and taken out therefrom, whereby silver-ionic water of pH 5 or less can be obtained. Here, the flow amount, voltage to be applied and so forth may be controlled to achieve the desired pH and silver ion concentration.

[Examples of the 2nd use]

<u>Example 2-1</u>

Soil improvement effect

Conditions:

Bare ground soil and greenhouse soil were prepared in amount of 100 g for each, and 100 cc each of two kinds of water (city water; electrical conductance:

187 $\mu\mho/cm^3$) and growth promotive acidic water (pH 3) of the present invention, produced with use of the city water, were respectively sprayed on, and mixed with, the soils. Thereafter, change of the water filtered was examined.

(1) Electrical conductance remaining in the soil:

pH 3 > city water

| Soil | Sprayed water | |
|---|---|---|
| | City water | pH 3 |
| Bare ground | 196 | 318 |
| Greenhouse | 196 | 328 |

Thus, the growth promotive acidic water of the present invention has high electrical conductance even after spraying in the soil, and can be expected to have the effect of compulsory substitution of metallic ions in the soil colloids.

(2) Change in pH:

| Soil | Sprayed water | | |
|---|---|---|---|
| | City water | pH 3 | pH 3 (after 1 hr) |
| Bare ground | 7.62 | 5.51 | 6.01 |
| Greenhouse | 7.87 | 5.98 | 6.42 |

Thus, in the case the growth promotive acidic water of the present invention is sprayed, the acidity is gradually buffered owing to the compulsory substitution in the soil colloids, giving less adverse effect to the growth of plants.

(3) The ppm of Ca ions: pH 3 > city water

| Soil | Sprayed water | |
|---|---|---|
| | City water | pH 3 |
| Bare ground | 44.2 | 92.8 |
| Greenhouse | 43.2 | 119.6 |

Thus, the growth promotive acidic water of the present invention has superiority in the effect of washing away the excessive metallic ions other than those substituted on the sound solid colloids.

(4) The ppm of Na ions: city water > pH 3

| Soil | Sprayed water | |
|---|---|---|
| | City water | pH 3 |
| Bare ground | 7.2 | 4.3 |
| Greenhouse | 7.7 | 4.2 |

In the case of Na ions, contrary to the case of Ca ions, the quantity of washed-away water becomes smaller when the growth promotive acidic water of the present invention is used. Since Na ions may be more readily washed away, there remains less excessive ions other than those substituted on the soil colloids. Accordingly, it can be said that the growth promotive acidic water of the present invention has a greater effect of washing away excessive metallic ions but it may little occur that the metallic ions adsorbed on the soil colloids are washed away together.

(5) Dissolved oxygen: pH 3 > city water

| Soil | Sprayed water | |
|---|---|---|
| | City water | pH 3 |
| Bare ground | 10.7 | 14.9 |
| Greenhouse | 10.9 | 16.5 |

Thus, te growth promotive acidic water of the present invention contains dissolved oxygen in a large quantity, and can supply sufficient oxygen necessary for the growth of plants.

(6) Filtration rate: (cc/min), pH 3 > city water

| Soil | Sprayed water | | | |
|---|---|---|---|---|
| | City water | | pH 3 | |
| | 1 hr | 2 hr | 1 hr | 2 hr |
| Bare ground | 25 | 30 | 39 | 39 |
| Greenhouse | 28 | 31 | 42 | 43 |

Thus, the growth promotive acidic water of the present invention has a greater filtration rate, which means that the effect by the substitution action can be obtained in a shorter time.

Example 2-2

Taking up 100 g of the soil for cultivation of tomatoes, the growth promotive acidic water of the present invention was added thereto and mixed to have the water content of 65 %, and the soil was collected after being left at 25°C for a given time to measure the number of bacteria contained in 1 cc of water. The growth promotive acidic water used had the pH of 5, silver ion concentration of 2,000 ppb, and electrical conductance of 1,500 $\mu U/cm^3$. Results obtained are shown in Fig. 6.

As will be clear from Fig. 6, the number of bacteria in the soil can be greatly decreased by adding the growth promotive acidic water of the present invention, but there is a tendency that the number of bacteria increases with

lapse of time.

Example 2-3

Tomato-harvesting effect:

Tomatoes, Bonderozer (name of plant), were cultivated by hydroponics in a glass room unheated. Medium used was rock wool and the cultivation was carried out according to a culture solution circulation system. As the culture solutions, an aqueous solution obtained by diluting an organic fertilizer "Bioaqua" (trademark; available from Sanraiku K.K.) to 1/1500 with the growth promotive acidic water of the present invention (ph 5; silver ion concentration: 2,000 ppb; electrical conductance: 400 $\mu \mho/cm^3$) was used as an example of the invention, and an aqueous solution obtained by diluting the organic fertilizer "Bioaqua" (trademark; available from Sanraiku K.K.) to 1/1500 with purified water was used as a comparative example.

Using these culture solutions and feeding water for 10 minutes every two hours in twenty-four hours, the number of mature berries, average days for maturation, and longest term for maturation of the tomatos thus cultivated were observed. Results obtained are shown in the following table.

(Properties of culture solutions)

| Properties | (Example) Bioaqua + silver-ionic water | (Comparative example) Bioaqua + silver-ionic water |
|---|---|---|
| pH | 6.5 | 7.19 |
| EC | 0.64 | 0.46 |
| mV | 638 | 342 |
| DO | 18.0 | 9.10 |

(Fruit-harvesting)

| | Number of mature berries | Average days for maturation (day) | Longest term for maturation (day) |
|---|---|---|---|
| Example | 57 | 50.1 | 54 |
| Comparative example | 44 | 58.5 | 73 |

As shown in the above tables, the maturation of the berries of tomatoes can be promoted by use of the growth promotive acidic water of the present invention, thereby achieving the effect of increasing the yield.

As described in the foregoing, according to the

present invention the silver-ionic water having the pH of 5 or less and silver ion concentration of 30 to 2,000 ppb is used in the cultivation of plants such as agricultural products, whereby the acidic state of greenhouse soil or the like due to the accumulation of salts can be remedied and a desired state of the soil colloids can be produced, so that pathogenic bacteria or the like can be effectively exterminated without care of any adverse effect that may be caused on human bodies by continuous application of agricultural chemicals, there can be produced a desired symbiotic environment that can propagate symbiotic useful bacteria in the soil can be produced, and also there can be obtained the effect of promoting the growth and maturation of fruit owing to the rise of auxin and ethylene.

A 3rd use of the silver-ionic water of the present invention is concerned with a plant growth promotive alkaline water for cultivation of agricultural products (hereinafter "growth promotive alkaline water", comprising silver-ionic water that can have effect such as soil-conditioning, controlling of pathogenic bacteria, promotion of the growth of foliage, promotion of the photosynthesis under a low amount of light, and so forth.

In cultivation of agricultural products, it is well

known that various types of soil-conditioning agents, agricultural chemicals and growth promotive agents have been used.

Under the weather conditions as in countries having abundant rainfalls throughout the year, there is also a tendency that the so-called leaching phenomenon occurs by which bases contained in the soil are dissolved to permeate into the lower part of the soil, and the soil may turn acidic. Once the soil has turned acidic, there may be caused various problems such that inorganic components necessary for plants become short, harmful ions such as Al ions increase, phosphoric acid is combined with aluminum or iron to become incapable of being absorbed, and further the activities of useful bacteria in the soil are weakened. For these reasons, countermeasures such as spraying of lime and application of organic fertilizers or phosphoric acid fertilizers have been often taken to prevent the soil from turning acidic.

However, the facts are that the spraying of lime or the application of organic fertilizers or phosphoric acid fertilizers may result in the decrease in fertility of the soil itself, may require greater labor and cost, and can not achieve remarkable effect in spite of them. Also, almost no positive effect has been attained in controlling pathogenic bacteria in the soil to increase the activities

of useful bacteria and prevent damage by diseases.

Also, in recent years, it has become very popular to carry out nutrient solution cultivation in a greenhouse, separated from the soil. The nutrient solution cultivation can be roughly classified into the mist culture, the hydroponics (or soilless culture) and the solid medium culture, all of which aim at factory production of agricultural products as they can artificially control the composition of culture solutions, and have an advantage that it is made possible to carry out the cultivation under the conditions better suited for crops.

However, in the nutrient solution cultivation in a greenhouse, the temperature and humidity tend to become higher to readily develop microorganisms such as harmful bacteria and harmful fungi in the culture solution, and, once the culture solution is contaminated, there is involved a fear of receiving a deadly blow in the harvesting of agricultural products. For this reason, it has been most questioned to prevent the damage by diseases, but there has been found no other way of solution than frequently using agricultural chemicals. However, as having been evidenced by a great number of cases, the frequent use of agricultural chemicals may bring about a potential danger of giving serious damage to

the health of humans. There is also a strong tendency that the use of agricultural chemicals for many years may cause development of bacteria resistant thereto, with a loss of the effect thereof.

In the cultivation of agricultural products, another important factor is the amount of light. Shortage of the amount of light may make the photosynthesis inactive to greatly decrease the growth rate. This is nearly a fate of the greenhouse cultivation, and has been considered to be a problem that can not be solved even with use of any sort of growth promotive agents.

Accordingly, a still further object of the present invention is to provide an epock-making growth promotive alkaline water that can solve the problems of the soil liable to turn acidic and age, can prevent damage by diseases on agricultural products without care of adverse effect to be caused on human bodies by the accumulation of chemical drugs in the plants such as agricultural products after continuous application of agricultural chemicals, can also have the action of promoting the growth of foliage of agricultural products, and further can have the action of enhancing the photosynthesis ability of agricultural products.

The growth promotive alkaline water of the present invention is characterized by comprising silver-ionic

water having the pH of 8 or more, silver ion concentration of 30 to 2,000 ppb, and electrical conductance of 250 to 450 $\mu\mho/cm^3$.

The growth promotive alkaline water of the present invention may be sprayed on the acidic soil, whereby the acidic soil can be neutralized by virtue of the alkalinity thereof, the inorganic components such as metals contained in the soil can be activated by being provided with increased electrochemical potential and forced to be substituted with hydrogen ions adhered on the soil colloids, and thus there can be formed well-balanced normal soil colloids abundantly containing inorganic components necessary for the growth of agricultural products.

In the soil cultivation, fertilization is often effected by using organic fertilizers together, and a large number of microorganisms, which may decompose organic substances in the soil, is present in the form of pathogenic bacteria, thick membrane spores or oospores. This may often result in such a state that even a strong agricultural chemicals may have no effect.

The growth promotive alkaline water of the present invention can be sprayed and permeated into the soil without any complex formation between proteins and silver ions contained in the soil, can come into contact with

pathogenic bacteria and other microorganisms as it is, can flow into the inside of cells through their cell membranes according to the inclination of the electrochemical potential, and can cause a phenomenon in which the cell membranes are made alcoholic by the catalytic action the silver ions have, whereby it is made possible to inactivate the function thereof and remarkably decrease the pathogenic bacteria and other microorganisms.

As a matter of course, the plant symbiotic useful bacteria may be also remarkably decreased, but, once thereafter the roots of plants begin to vigorously grow and begin to secrete organic substances, only the symbiotic useful bacteria may greatly propagate themselves to produce a sound symbiotic environment for plants. Here is made the most of the time difference before the harmful microorganisms again propagate themselves destroying the stable symbiotic relationship.

In the case of the nutrient solution cultivation, the growth promotive alkaline water may also be mixed into the nutrient solution, whereby the action to the organic fertilizers or the cell membranes of bacteria or fungi adhered to proteins in the roots, and also the effect of inactivating them, can be achieved in the entirely same fashion.

The growth promotive alkaline water of the present

invention may also be sprayed on foliage of plants, to obtain the equally remarkable bacteriostatic effect against pathogenic bacteria on the foliage.

The silver-ionic water of the present invention is harmless to human bodies, and free from such dangerousness as the agricultural chemicals may have. Moreover, it is advantageous that the bacteriostatic effect achievable by the silver-ionic water have nothing to do with the generation of resistant bacteria.

The growth promotive alkaline water of the present invention has a remarkable effect in promoting the growth of foliage of plants, particularly, vegetables, and can have the effect of harvesting in a short time and increasing the yield.

A great number of tests has proved that hormones formed in the plant living bodies are greatly concerned with the growth of plants. A considerable number of cases can be seen in which the cultivation has been promoted by chemically synthesized hormone substances.

Typical growth hormones may include, as having mentioned herein, auxin, gibberellin, cytokinin, ethylene, etc. Of these, gebberellin is known as a hormone participating in the promotion of the growth of foliage.

Although in many fields it has not been made clear what a share a plant hormone has in cell walls and cells,

a verification test has recognized a phenomenon that auxin participates in the softening of cell walls to enhance the permeability and reversible extension of wall membranes, and gibberellin increases the osmotic pressure potential of cell liquids to promote the growth of the cells.

It is further known that gibberellin is activated depending on the degree of saturation in a suitable substituted A-ring having an elementary gibberellin carbon skeleton, and the number and position of hydroxyl groups (-OH).

Standing on this phenomenon, the present inventors have assumed that silver ions may be participated as a catalyst, whereby $C_2H_5$ of ethyl indoleacetate which is a form of the storage of auxin in plant cells may be separated as $C_2H_4OH$ to inhibit the effect of ethylene, while, at the same time, auxin may be activated to proceed with the softening of cell walls, and also a large quantity of the hydroxyl groups weakly combined with silver ions may be supplied to the elementary structure having the gibberellin carbon skeleton, whereby the activation of gibberellin can be promoted, the osmotic potential of cell liquids can be increased, and thus the foliage can abruptly grow by the absorptive action of water having been ionized in a large quantity, and further that, at the root portions, the relative concentration of

auxin and cytokinin reaches a given amount, whereby there can be seen extension of roots and increase in the absorption rate.

Then, silver-ionic water having the pH of 8 or more, silver ion concentration of 30 to 2,000 ppb, and electrical conductance of 250 to 450 $\mu\mho/cm^3$ was supplied. As a result, the acidic soil was neutralized, the soil colloids were also normalized, and the osmotic pressure in the soil was lowered than the osmotic pressure of the roots, whereby it was made possible that the absorption potential of water was increased, the auxin in the plant living bodies was activated by virtue of the catalytic action of silver, and the gibberellin was activated by supplying of $OH^-$ ions. As a result, as shown in Example 3-3, the root portions greatly extended to achieve surprising extension in a short time with increase by 20 to 30 % of usual cases, successfully.

The growth promotive alkaline water of the present invention has further made it possible to achieve the growth of foliage of agricultural products at a lower amount of light. The mechanism of this invention is considered as follows:

Chloroplasts (chlorophyl) of plants utilize light energy (hr) and utilize hydrogen in water as a reducing agent to effect fixation reduction of $CO_2$ to achieve the

conversion of carbohydrates, in the course of which ATP is synthesized. There are two reactions, one of which is the light reaction by which NADP is reduced ($2H_2O$ + 2NADP ---> xADP + xP light ---> $2NADPH_2$ + xATP + $O_2$) and the dark reaction called Calvin-Benson cycle by which $CO_2$ is fixed to carbohydrates by ATP and $NADPH_2$ ($CO_2$ + $2NADPH_2$ + xATP ---> $CH_2O$ + $H_2O$ + 2NADP + xADP + xP). In the course of these reactions, there are a phenomenon that $H^+$ ions are incorporated into chloroplasts to lower the pH in the inside of thylakoid membranes when chloroplasts are exposed to light, and a phenomenon that $H^+$ ions, $OH^-$ ions and e' (electrons) participate in the formation of $NADPH_2$ and synthesis of ATP. From these phenomena, if the light energy is converted to the difference in the electrochemical potential of $H^+$ ions and this is used for the synthesis of ATP, it should follow that the synthesis of ATP can be effected by artificially imparting the difference in the electrochemical potential of $H^+$ ions to the inside and outside of the membranes of chloroplasts. It is further considered to be possible that auxin and gibberellin which are each a sort of growth hormones as mentioned above can be activated by supplying Ag ions and $OH^-$ ions, thereby allowing foliage to sufficiently grow even at a lower amount of light.

In this connection, experiments were carried out to

examine the possibility of the formation of ATP by an acid-base treatment on chloroplasts.

In order to introduce $H^+$ ions to the inside of chloroplasts, chloroplasts were dipped for one minutes in a reaction mixture of pH 4 to which an electron transport inhibitor DCMU was added to show that no electron transport system was participated, and the treatment was carried out in a dark room. Then, prepared was a reaction mixture to which ADP and $^{32}P_1$ and further $Mg^{2+}$ necessary for the synthesis of ATP were added, which was made alkaline by addition of tris NaOH, and the chloroplasts dipped in the above reaction mixture of pH 4 were put into it. In this manner, the so-called acid-base treatment was completed in the state that the external liquid was alkaline but the inside of chloroplasts remained acidic. It was proved that ATP is produced according to the resulting difference in the electrochemical potential between the inside and outside of the cells. Now, using an apparatus (mentioned herein) for producing silver-ionic water, prepared was silver-ionic water having the pH of 8 or more, silver ion concentration of 30 to 2,000 ppb, and electrical conductance of 250 to 450 $\mu$U/$cm^3$, which was sprayed on the soil or mixed in the nutrient cultivation water, and allowed to be absorbed in plants from the roots thereof. As a result of the experiment (as shown in

Example 3-4), the foliage was excellently grown successfully, even at such a lower amount of light that no growth of plants could not be expected in usual case.

The silver-ionic water constituting the growth promotive alkaline water of the present invention is made to have the pH of 8 or more, and silver ion concentration of 30 to 2,000 ppb.

The reason why the pH is made to be 8 or more is that the presence of $H^+$ protons greatly participates in the combination between trace elements and proteins, without regard to the kind of the trace elements, and, under the conditions of the pH of more than 5 and less than 8, silver ions tend to form complexes with organic substances such as proteins in the soil or nutrient solution and be readily absorbed, whereupon there can not be exhibited any bacteriostatic effect or physiological activity effect the silver ions may have.

The reason why the silver ion concentration is made to be 30 to 2,000 ppb is that the concentration of less than 30 ppb may result in no bacteriostatic effect in the soil or nutrient solution, and the concentration more than 2,000 ppb may result in the inhibition of even the growth of useful bacteria such as symbiotic bacteria.

In a preferred embodiment of this 3rd use of the silver-ionic water of the present invention, the growth

promotive alkaline water is made to have electric conductance of 250 to 450 $\mu$Ʊ/$cm^3$. The electric conductance made to be 250 $\mu$Ʊ/$cm^3$ or more may increase electrochemical potential, whereby silver ions are made to readily permeate cell membranes of bacteria or the like, and further the hydrogen ions combined with soil colloids can be forced to be substituted with metallic ions. The electric conductance less than 250 $\mu$Ʊ/$cm^3$ may result in poorness of the above effect, and the electric conductance more than 450 $\mu$Ʊ/$cm^3$ may result in difficulty in the production.

The growth promotive alkaline water of the present invention can be produced by the apparatus shown in Fig. 1.

Namely, using the apparatus, water may be passed through the first electrolytic cell chamber with application of voltage, where silver ions are dissolved out, and the water thus treated may be passed through the cathode compartment of the second electrolytic cell chamber and taken out therefrom, whereby alkaline silver-ionic water of pH 8 or more can be obtained. Here, the flow amount, voltage to be applied and so forth may be controlled to achieve the desired pH and silver ion concentration.

[Examples of the 3rd use]

Example 3-1

Conditions:

Bare ground soil and greenhouse soil were prepared in amount of 100 g for each, and 100 cc each of two kinds of water (city water; electrical conductance: 187 $\mu U/cm^3$) and growth promotive alkaline water (pH 10; electrical conductance: 304 $\mu U/cm^3$) of the present invention, produced with use of the city water, were respectively sprayed on, and mixed with, the soils. Thereafter, change of the water filtered was examined.

(1) Electrical conductance remaining in the soil:

City water > pH 10

| Soil | Sprayed water | |
|---|---|---|
| | City water | pH 10 |
| Bare ground | 196 | 123 |
| Greenhouse | 196 | 126 |

The growth promotive alkaline water of the present invention has lower electrical conductance. This means that $H^+$ ions in the soil colloids are combined with $OH^-$ ions in the growth promotive alkaline water to form hydrated ions and neutralize the water.

(2) Change in pH:

| Soil | Sprayed water | |
|---|---|---|
| | City water | pH 10 |
| Bare ground | 7.621 | 8.23 |
| Greenhouse | 7.87 | 8.83 |

The growth promotive alkaline water of the present invention has a high pH as shown above, and can be expected to have the effect of neutralizing the acidic soil.

(3) The ppm of Ca ion: City water > pH 10

| Soil | Sprayed water | |
|---|---|---|
| | City water | pH 10 |
| Bare ground | 44.4 | 28.7 |
| Greenhouse | 43.2 | 36.9 |

The growth promotive alkaline water of the present invention shows a lower ion concentration, but this is presumably because $H^+$ ions adhered on the soil colloids have been substituted with Ca ions, which Ca ions have been adsorbed on the soil colloids.

(4) The ppm of Na ions: pH 10 > city water

| Soil | Sprayed water | |
|---|---|---|
| | City water | pH 10 |
| Bare ground | 7.2 | 28.7 |
| Greenhouse | 7.7 | 36.9 |

The growth promotive alkaline water of the present invention shows a lower Na ion concentration, but this is presumably because excessive Na ions have been leached away.

Example 3-2

Bactericidal test

In general, viruses are said to be inactivated at pH 4.5 or less and pH 10.5 or more. They are also said to be inactivated by ultraviolet light of 253.7 nm. Now, tests were carried out to compare the effect by silver-ionic water of pH 8.5, silver ionic water of pH 10.5 and the ultraviolet irradiation.

Conditions:

1) Bacterial body: Tobacco mosaic virus

2) Inoculation:

Tobacco mosaic viruses were added to aluminum oxide powder, water was added thereto, and the mixture formed was sprayed, thereafter softly rubbing the

leaf surface for plantation.

3) Seedling culture temperature: 25°C

4) Spraying of silver-ionic water:

Each of the above silver-ionic water was sprayed 24 hours after inoculation. Ultraviolet irradiation was also carried out.

5) Measurement:

The number of diseased spots was measured on 10th day after inoculation, and an average of the spots included in one plant.

Results obtained by the measurement are shown in Fig. 7 and Fig. 8. In Fig. 7, curve "a" shows the result obtained by spraying silver-ionic water of pH 8.5 in concentration of 1 ppm; curve "b", the result obtained by spraying silver-ionic water of pH 8.5 in concentration of 2 ppm; and curve "c", the result obtained by spraying silver-ionic water of pH 10.5. Fig. 8 shows the result obtained by ultraviolet irradiation, wherein the ultraviolet light had wavelength of 253.7 nm and irradiated at a dose of 7,000 $erg/mm^2/min$.

Example 3-3

Using ordinary bare ground soil, stone parsely (*Crytotaenia japonica*) was cultivated in the following three-divided quadrats for each 5 plants.

(1) Silver-ionic water-treated quadrat:

As the culture solution, an aqueous solution obtained by diluting an organic fertilizer "Bioaqua" (trademark; available from Sanraiku K.K.) to 1/2000 with the growth promotive alkaline water of ph 8.5, silver ion concentration of 2,000 ppb and electrical conductance of 350 $\mu U/cm^3$ was used.

(2) Ionic water-treated quadrat:

As the culture solution, an aqueous solution obtained by diluting an organic fertilizer "Bioaqua" (trademark; available from Sanraiku K.K.) to 1/2000 with ionic water obtained by electrolysis of general city water to have the pH of 8.5 was used.

(3) Control:

As the culture solution, an aqueous solution obtained by diluting an organic fertilizer "Bioaqua" (trademark; available from Sanraiku K.K.) to 1/2000 with purified water.

These culture solutions were supplied by showering for 20 minutes at 8 o'clock every day, and the degree of growth in each quadrat was measured. Results obtained are shown in Table 9.

It is seen from Table 9 that the growth of stems and leaves was clearly promoted when the growth promotive alkaline water of the present invention was used.

Table 9

| Quadrat | When treated | | After 2 weeks | | | | | |
|---|---|---|---|---|---|---|---|---|
| | Number of leaf | Length of leaf | Number of leaf | Length of leaf | Width of leaf | Length of petiole | Living weight | Figure of plant |
| | (leaf) | (cm) | (leaf) | (cm) | (cm) | (cm) | (g) | |
| (1) | 6.8 | 14.5 | 10.3 | 23.0 | 5.5 | 11.5 | 5 | Grown in uniform figure |
| (2) | 6.1 | 13.8 | 8.0 | 19.0 | 4.1 | 8.7 | 4.2 | Grown in not unform figure |
| (3) | 6.5 | 14.3 | 8.5 | 18.5 | 4.3 | 9.1 | 4.1 | Grown in not unform figure |

Example 3-4

Radishes Kaiware were cultivated under the following conditions to examine the germination rate, yield, and state of the rot of roots.

Cultivation room: Three wooden culture rooms of 50 cm wide, 2 m and 50 cm long, and 1 m high each were prepared.

Light source: Using one electric lamp of 40 W x 100 V, irradiated with 1100 lux.

Medium: Urethane cotton.

Water supply:

Using the following culture solutions A, B and C, an immersion system was employed in respect of culture solutions B and C, and the two of an immersion system and a shower system were employed in respect of culture solution A. The immersion system is a method in which a medium is immersed in the culture solution to carry out the cultivation, and the shower system is a method in which the culture solution is supplied by a shower bath at every 2 hours and 30 minutes to carry out the cultivation.

Culture solutions:

(A) Growth promotive alkaline water of pH 8.5, silver ion concentration of 2,000 ppb, and electrical conductance of 350 $\mu℧/cm^3$.

(B) Ionic water of pH 8.5 and electric conductance of 250 µU/$cm^3$, obtained by electrolysis of general city water.

(C) General city water.

Results of this experiment are shown in Table 10.

Table 10

| Culture solution (Water supply system) | Amount of light (lux) | Germination rate (%) | Yield (g) | Rot of roots |
|---|---|---|---|---|
| (C) General city water (Immersion) | 1100 | 75 | 750 | Found pulpy |
| (B) Alkali-ionic water (Immersion) | 1100 | 75 | 850 | Found pulpy |
| (A) Silver-ionic water (Immersion) | 1100 | 96 | 1100 | None |
| (A) Silver-ionic water (Shower) | 1100 | 95 | 1100 | None |

It is seen from Table 10 that the germination rate is higher, the yield is larger and the root rot has been prevented when the growth promotive alkaline water of the present invention is used.

As described in the foregoing, according to the present invention the silver-ionic water having the pH of 8 or more and silver ion concentration of 30 to 2,000 ppb is used in the cultivation of agricultural products, whereby the acidic soil can be neutralized to form soil colloids well-balanced and rich in the nutrients such as inorganic components, the damage by diseases can be effectively prevented without care of any adverse effect on human bodies, and the propagation of useful bacteria such as symbiotic bacteria in the soil can be promoted to produced a desired symbiotic environment.

Moreover, by virtue of the physiological activities and action exerted by the silver-ionic water, the growth of foliage of agricultural products can be promoted, and also the ability for the photosynthesis can be enhanced, whereby it is made possible to gain a high yield even in an environment short in the amount of light.

A 4th use of the silver-ionic water of the present invention is concerned with a therapeutic water for

pathogenobacterial skin diseases and external cavity mucosal diseases such as skin ecphyma disease, tonsillitis, pyorrhea alveolaris, trichosis disease, gangrenous disease and anal fistula disease, which are caused by infection of human skin or external cavity mucosa with pathogenic microbes such as bacteria.

Recent years, it is true that rapid strides in the development of antibiotic substances, antibacterial agents, chemotherapeutic agents and so forth have realized high grade medical treatments for patients infected with pathogenic microbes such as bacteria, regardless of medicine or surgery. Also, the therapy that has ever been done by surgical operations can be performed by medicinal treatments, and there are many cases in which sufficient cure has been achieved. However, on the other hand, there can be seen cases where a large amount administration of broad spectrum antibiotics has caused fixation of resistant bacteria to organs to have brought about inviterate infectious diseases. Namely, the state of things is such that there are gradually increasing special diseases or strange infectious diseases such as opportunistic infectious disease, bacterial substitution disease and nosocomial infectious disease.

Also, most of the chemotherapeutic agents nowadays used may achieve their pharmacological action by giving a

blow to a living body and damaging the same. Although living humans in the present generation may be safe in view of the safety degree according to $LD_{50}/ED_{50}$, there is no guarantee on the next generation or the heritability.

From the viewpoints as above, as therapy for pathogenobacterial skin diseases and external cavity mucosal diseases caused by infection of human skin or external cavity mucosa with pathogenic microbes such as bacteria it has been sought after to discover therapy based on a new function or mechanism, replacing the present chemotherapeutic agents.

Accordingly, a still further object of the present invention is to provide a novel therapeutic water for pathogenobacterial skin diseases and external cavity mucosal diseases. The present invention also aims at providing therapeutic water for pathogenobacterial skin diseases and external cavity mucosal diseases, that can have a bacteriostatic or bactericidal action on pathogenic bacteria according to the mechanism in the natural life system, and also can promote the mechanism of the recovery of organism cells. The present invention further aims at providing therapeutic water for pathogenobacterial skin diseases and external cavity mucosal diseases, that can have simultaneously the three functions of i) bacteriostatic or bactericidal action on pathogenic

bacteria, ii) decomposing and absorbing destroyed tissue and iii) of immediately regenerating neogenetic tissue, and also can be iv) odorless, v) colorless, vi) promptly effective and vii) simple.

The above objects can be achieved by therapeutic water for pathogenobacterial skin diseases and external cavity mucosal diseases, comprising silver-ionic water of pH 5 or less and silver ion concentration of 0.5 to 10 ppm.

In a preferred embodiment of the 4th use of the present invention, the therapeutic water may have electrical conductance of 350 to 2,000 $\mu℧/cm^3$, and a dissolved oxygen amount of 12 to 30 ppm.

The therapeutic water for pathogenobacterial skin diseases and external cavity mucosal diseases (hereinafter referred to merely as "therapeutic water") of the present invention is based on a mechanism entirely different from the conventional therapeutic agents. Specifically, the conventional therapeutic agents may show pharmaceutical effect by giving to living bodies the inhibitory effect such as i) inhibition of cell wall synthesis, ii) inhibition to cell membranes, iii) inhibition of protein synthesis or iv) inhibition of the synthesis of nucleic acid and DNA, and nothing has been available that can expect therapeutic effect in the mechanism in the natural

life system. In particular, in respect of the inhibition of cell membranes, which are mostly common to animal cells, there may be readily accompanied with side effect to require an extreme limitation in use. On account of these, the present inventors have analyzed the rise and fall of cell in the living bodies, and thereby discovered for the first time the mechanism of artificial bacteriostatic or bactericidal action in the natural life system.

Of the cells composing a body, some live as long as it can live once they have been completed, some repeat new formation and destruction by themselves, and there are some other various ones, but, observing the manner of the death of cells, a change often occurs firstly in the nucleus. The change occurring in the nucleus is the concentration of chromosomes, and, in contrast thereto, diffusion takes place in cytoplasms due to the inflow of water from outside. It can be said that a cell, wherein a cell membrane controls the going in and out of substances, is living while the membrane functions positively, and dead when the function is lost. As a result of inflow of water to the cytoplasm, a structure of the membrane may be vacuolated and may be soon destroyed to lose its shape, whereupon small organs also may be thrown out and the cell come to death. However, even with some damage applied to

cytoplasma, the cell can repair oneself if the nucleus can sufficiently function. To analyze this mechanism, the object can be achieved if the function of cell membranes can be artificially inactivated for a certain period, in other words, inactivated by a special catalytic function to the composition forming the membranes of cells, but, on the other hand, a prerequisite is that no adverse effect should be given to living bodies. As a result of a great number of experiments and verification having been hitherto made, the present inventors have succeeded in inactivating target bacteria by utilizing the mechanism possessed by silver ions acting only on 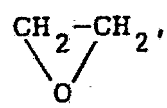, to catalyze unsaturated aliphatic acids of phospholipids constituting the cell membranes for the formation thereof into ethylene. Moreover, they have succeeded in freely revive the target bacteria by controlling ion concentration, even once they have been made static. In order to feed silver ions into the bodies of pathogenic bacteria in the co-presence of proteins, it is of course necessary to prevent the action of silver ions being lowered because of the complex-forming combination with proteins on the way of the feeding. For that purpose, there may be produced a condition of pH 5 or less that may not cause the complex-forming combination, and may be

provided with electrochemical potential so that, even if cell bodies may act to take the form of spores, the silver ions may invade cells by utilizing the difference in the potential gradient. Further, the $LD_{50}/ED_{50}$ to know the influence to normal human cells is desired to be as high as possible. However, since the safety value of silver ions which has been established by experiments and verification is 500 ppm and the concentration necessary for killing bacteria, of the therapeutic water of the present invention is 0.5 to 10 ppm, there is nearly almost no harm to human bodies, which could not be expected at all in the conventional chemical drugs.

The therapeutic water according the 4th use of the present invention will be described below in detail based on its specific action.

All the cells, ranging from animal cells to pathogenic microbes such as bacteria, fungi, viruses, protozoa, are enveloped with cell membranes, having an inseparable relationship between these membranes and cell bodies, and life can not exist unless membranes exist. These membranes of cell bodies are sources of fundamental activities in every kind of life phenomena, and constituted with both natural order and national mechanism, though complicated as in concentration or potential difference of substances present inside or

outside of the cells, difference in the manner of existence, counterattacks in other cells, etc.

The cell membranes have the structure of double membranes comprising phospholipids. Of the phospholipids, phosphatidyl serine, phosphatidyl ethanolamine and phosphatidyl inositol are principally present in the inside of membranes, and, on the other hand, phosphatidyl choline is mostly present at the outside of membranes.

Membrane-combinable proteins which combine with these cell body membrane can mostly show physiological activities including enzymatic activity, and are well known to contain receptors thereof or carrier proteins participating in ion channels and transport of substances.

Having analyzed the substance-constitution of cells, there are contained 25 to 60 % of polar lipids to construct the fundamental structure with lipid double layers and membrane proteins. The phospholipids contained therein are rich in readily oxidizable polyvalent unsaturated aliphatic acids. In the polar lipid molecules, hydrophilic part is in direct contact with an aqueous phase and has electric resistance of as low as $10^2$ to $10^5$ $\Omega/cm^3$. On the other hand, hydrophobic part contains a large quantity of $-CH_2-$ such as aliphatic acids, which are arranged in parallel to each other by hydrophobic bond to form double structure. These may have

significant meaning for the development of pathogenic beds.

Here, inactivation of cells, when viewing the manner in which cell membranes themselves are inactivated even in a natural environment, as cell membranes actually participate therein, radicals of the polyvalent unsaturated aliphatic acids are formed when peripheral ion type substances or OH groups are formed in the vicinity of membranes, and combine with biradical oxygen to form aliphatic acid peroxyradicals to successively cause hydrolysis, and finally the membranes are broken. In order to artificially cause this mechanism to bring about to inactivate target bacteria, a catalyst capable of causing these chemical reactions is required, and there can be found nothing other than silver as the substance having such functions. The catalytic action of the silver, that can form $C_2H_4O$ as a partially oxidized product in the oxidation reaction of ethylene, is a selective action that no other metallic ion can achieve. Accordingly, the therapeutic water of the present invention contain the silver having the above action as silver ions in concentration of 0.5 to 10 ppm, more preferably 1 to 7 ppm, most preferably 3 to 6 ppm.

The silver ions in the therapeutic water of the present invention can pass through cell membranes (and

also cell walls) having the function of separating and passing proteins comprising giant molecular colloidal particles from a liquid, enter the bacterial bodies, and inactivate the function of cell membranes by virtue of the selective function pertaining to silver ions, i.e., the catalytic action for 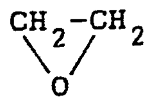. The experiments made by the present inventors revealed that the bacterial bodies once made bacteriostatic again began to regenerate and propagate themselves at the silver ion concentration of about 100 to 500 ppb, the regeneration rate was lowered with increase in the concentration, and there resulted in an almost sterilized state at the concentration of 0.5 to 10 ppm.

Also, in respect of human cells, whose safety degree of $LD_{50}/ED_{50}$ is 50 ppm for healthy normal cells, a noticeable phenomenon has been discovered, that cells attacked by pathogenic bacteria may follow substantially the same tendency as that for cell bodies. Accordingly, treatment by the therapeutic water of the present invention can inactivate pathogenic bacteria and, at the same time, break the tissue affected by pathogenic bacteria, and also makes it possible to eliminate the inhibition of regeneration of tissue by such affected tissue. On the other hand, no adverse effect was given to

the cells of normal tissue, and there was seen no problem even at mucosal portions having weaker resistance as compared with skin.

The therapeutic water of the present invention may have the pH value of pH 5 or less, more preferably pH 4 or less, and most preferably pH 3 or less. Making the therapeutic water to have the pH value of 5 or less as above, adsorption of silver ions on proteins can be prevented, a lowering of the catalytic action of silver ions can be prevented even under the conditions of co-presence of proteins, and it is made possible for silver ions to freely permeate the cell membranes of target bacterial bodies.

The therapeutic water of the present invention may further have electrical conductance of 350 to 2,000 $\mu\mho/cm^3$, more preferably 1,000 $\mu\mho/cm^3$ or more, and most preferably 1,500 $\mu\mho/cm^3$ or more. Making the therapeutic water to have the electrical conductance of 350 to 2,000 $\mu\mho/cm^3$, the cell permeability of silver becomes better, and it is made possible for the action of silver ions to be sufficiently exhibited.

In addition, the therapeutic water of the present invention may be made to have a dissolved oxygen amount of 12 to 30 ppm to provide a desirable environment for the cure and regeneration of diseased tissue.

The present inventors produced silver-ionic water of pH 5 or less, silver ion concentration of 0.5 to 10 ppm, electrical conductance of 300 to 2,000 $\mu U/cm^3$ and a dissolved oxygen amount of 12 to 30 ppm in the manner as described below, and first dip-applied to the part affected by a pathogenobacterial (yellow Staphylocuccus) darmatosis. As a result, there was brought about a dramatical effect that could not have been seen in the conventional chemotherapeutic agents. Then, they further applied it to diseases such as scale disease on head, gangrene, gonorrhea, pyorrhea and athlete's foot. As a result, regardless of the kind of pathogenic bacterial bodies in the affected part, the bacteria were killed in a day or so in all the cases, pains were removed immediately after therapy, hemostatic effect were brought about, injured bacteria were decomposed and absorbed to start regeneration of sound cells only, surprising recovery was shown by virtue of, e.g., the supply of activated oxygen, and there were achieved therapeutic effect such that there was no difficulty even if the cured part was touched or bent and stretched.

The production of therapeutic water of the present invention can be preferably carried out without formation of silver colloids, by use of the above apparatus shown in Fig. 1.

[Examples of the 4th use]

Example 4-1

Symptom: Statis eczema

On lower extremities having varicosis, flushes were strongly madidans. Part thereof was affected by self-infectious dermatitis.

Treatment:

Silver-ionic water of pH 3, silver ion concentration of 3 ppm, electrical conductance of 1,000 $\mu U/cm^3$ and a dissolved oxygen amount of 15 ppm was applied on the affected part, and thereafter a wet compress with the silver-ionic water was applied thereon for 30 minutes.

Effect:

After therapy, itchiness was removed in 5 minutes, flushness was removed after 30 minutes, and normal skin was recovered in a day.

Example 4-2

Symptom: Acute prurigo infant strophulus

Erythemas and pomphuses were seen, and small blisters due to scratching were seen on extremities and trunks.

Treatment:

Silver-ionic water of pH 3, silver ion concentration of 3 ppm, electrical conductance of 1,000 $\mu U/cm^3$ and a dissolved oxygen amount of 15 ppm was sprayed on the whole

body, and thereafter a cold wet compress with the same water was applied.

Effect:

Itchiness and pain were removed about 10 minutes after therapy, pomphuses were gone down after 24 hours, traces of scratching on the skin were also almost disappeared, and the affected part was perfectly cured.

Example 4-3

Symptom: Pustular psoriasis

State of erithroderma and mucutitis was seen. Stomatitis was also complicated. Squamouses were seen on the skin of hands.

Treatment:

Silver-ionic water of pH 4, silver ion concentration of 4 ppm, electrical conductance of 800 $\mu U/cm^3$ and a dissolved oxygen amount of 20 ppm was sprayed, and thereafter a wet compress with the silver-ionic water was applied. Regarding the oral cavity, the same silver-ionic water was offered for mouth-washing with instructions of mouth-washing three times a day.

Effect:

Mucutitis was cured in a day, the state of erithroderma became faint after three days to recover normal skin. Squamouses were perfectly cured in 12 hours.

Example 4-4

Symptom: Folliculitis deculvans

Empyeses were produced in hair follicles, and spread one after another to adjacent hair follicles. Bacteria: Sporotrichosis.

Treatment:

Silver-ionic water of pH 4, silver ion concentration of 4 ppm, electrical conductance of 800 $\mu$U/$cm^3$ and a dissolved oxygen amount of 20 ppm was sprayed with use of a spray gun, and the skin was massaged.

Effect:

Dandruff-like cicatrixes were removed a day after therapy, and, 3 days after, the affected part was in almost perfectly cured state with spraying three times.

Example 4-5

Symptom: Pomphus simplex

Small blisters accompanied with red areolae were broken out in a continuous fashion, showing an erosive state in part. Viral disease.

Treatment:

A wet compress was impregnated with silver-ionic water of pH 4, silver ion concentration of 4 ppm, electrical conductance of 800 $\mu$U/$cm^3$ and a dissolved oxygen amount of 20 ppm, and applied with changing several times for each about one hour.

Effect:

Tingly feeling was removed 5 minutes after treatment, blisters were almost gone down after 24 hours, and the tension of skin surface was also recovered. No recurrence was seen thereafter.

Example 4-6

Symptom: Condyloma acuminatum

Affected papules were seen at labial periphery, with their pultaceous surfaces having strongly rank odors, complicated with hard chancre. Viral disease.

Treatment:

Silver-ionic water of pH 4, silver ion concentration of 4 ppm, electrical conductance of 800 $\mu$U/$cm^3$ and a dissolved oxygen amount of 20 ppm was filled in a water tray, and the affected part was dipped therein for 2 hours.

Effect:

Rank odor was lost after 2 hours, and the pain due to the friction by maceration was removed. After 3 day attendance at hospital, superficial symptom was gone, scars fell away, and hard chancre was perfectly cured in 5 days.

Example 4-7

Symptom: Foot ringwarm (athlete's foot)

The skin at toe crotches, particularly 4th toe crotch, underwent maceration, having radish erosion and

rhagadeses. Trichophyton.

Treatment:

Silver-ionic water of pH 2.5, silver ion concentration of 5 ppm, electrical conductance of 1500 $\mu\mho$ /$cm^3$ and a dissolved oxygen amount of 15 ppm was poured in a water bucket, and feet were dipped in it for 30 minutes in the total in one hour. Thereafter, a wet compress with the silver-ionic water was applied, which was stopped after 4 hours.

Effect:

The pain on vola was perfectly removed one hour after the dipping, rhagadeses were close up after 24 hours to form white collagen-like proud flesh and curticle, and no recurrence was seen after a wet compress was continually applied for 3 days.

<u>Example 4-8</u>

Symptom: Pyorrhea alveolaris (Marginal periodontitis)

Conjection and gingivitis occurred in succession because of mixed bacterial infection at periodontal pockets, and bleeding was seen in part, with strong halitosis.

Treatment:

Using silver-ionic water of pH 3, silver ion concentration of 4 ppm, electrical conductance of 1100

$\mu U/cm^3$ and a dissolved oxygen amount of 12 ppm, mouth-washing was made. Thereafter, cotton impregnated with the same silver-ionic water was carefully attached to the periphery of gingiva, and mouth-washing was carried out rigidly. The same silver-ionic water for a day was handed as mouth-washing water.

Effect:

Conjection was diminished 30 minutes after the treatment, halitosis was also removed, and the bleeding stopped. Next day, after lapse of 24 hours, an examination on the ambulant patient revealed that halitosis was removed, no edema was found, and the symptom of periodontitis had disappeared.

As described in the foregoing, the therapeutic water for pathogenobacterial skin diseases and external cavity mucosal diseases according to the present invention, which comprises silver-ionic water of pH 5 or less, silver ion concentration of 0.5 to 10 ppm, can achieve therapy based on action and mechanism that the three types of mechanism such that bacteriostatic or bactericidal action can be had on every kind of bacteria that may be widely concerned with the antibacterial spectrum, broken tissue can be decomposed and absorbed, and new organs can be immediately regenerated, are simultaneously realized, which is

entirely different from the conventional chemotherapic agents. It is excellent therapeutic water that can also satisfy the requirements for odorlessness, colorlessness, immediate effectiveness and readiness, and can be preferably used for the therapy of pathogenobacterial skin diseases and external cavity mucosal diseases such as skin ecphyma disease, tonsillitis, pyorrhea alveolaris, trichosis disease, gangrenous disease and anal fistula disease, which are caused by infection of human skin or external cavity mucosa with pathogenic microbes such as bacteria.

A 5th use of the silver-ionic water of the present invention is concerned with a wound-curative water used for cure of wounds and for surgeries, that can facilitate removal of pain at wounded part caused by injuries or surgeries; hemostasis, prevention of infectious diseases, and promotion of the recovery of tissue.

The wounds refer to a phenomenon in which an abnormal state has occurred in body tissue because of injuries or an operation itself by hands of a surgeon, which is an unsound state brought about by interruption of normal physiological and morphological connection in tissue. In such a state, there occur pain and bleeding, and also is involved in a danger to cause infectious

diseases caused by pathogenic bacteria, so that it is desired to achieve an early recovery of tissue by suitable treatment.

Firstly, the pain a patient may feel from a wound has various types depending on the range and degree of wounded cell tissue. Known methods of removing the pain include a method in which analgesics are used, and a method in which a pain-transmitting rout is cut at a suitable portion. However, these methods may only provide a temporary effect, or may give a large load to, and be highly dangerous for, patients. From these viewpoints, to achieve favorable recovery after surgery taking account of the influence to mentality of patients, development has been sought after to provide a harmless, high grade medical technique that can substitute the conventional analgesic method such as the temporary anesthesia using drugs or the cutting of pain-transmitting route in surgeries, and can remove the source of pain by analyzing the mechanism of inhibition in the life system.

A problem in the injuries or the surgeries may include the bleeding. The bleeding is grouped in two types, one of which is the bleeding from blood vessels and the other of which is the exudation from blood cell walls that is operated as a mechanism for natural cure. The hemostasis to cope with such bleeding, in particular,

hemostasis in operations, is a very important, fundamental work in surgeries. If the hemostasis is not carried out surely, it would be difficult to expect success in the operation, and, in the case of an intracraial operation, unsure hemostasis may directly lead to death. Further, in case of insufficient hemostasis, there is a fear of forming blood tumors or sanious pus even after the operation, which may turn to dead cavities to cause infectious diseases. The more detailed the operation is, the more necessary it is to provide a means for stop bleeding from minute vessels, but there has been developed no radical technique.

One of other great problems which may arise after operation or in the course of cure process is an infectious disease affected by pathogenic bodies such as bacteria, fungi, viruses and protozoa. In high level surgeries or cure of heavy wounds, for which broad spectrum antibiotics may be administered in a large quantity for preventing the infectious diseases, it may occur that resistant bacteria are fixed to tissue to cause an advance to intractable infectious diseases, for example, opportunistic infectious diseases. Nowadays, there is a trend of increase in such cases.

Accordingly, a still further object of the present invention is to provide a wound-curative water used for

cure of wounds and for surgeries, that can facilitate removal of pain at wounded part caused by injuries or surgeries; hemostasis, prevention of infectious diseases, and promotion of the recovery of tissue. The present invention also aims at providing a wound-curative water that can facilitate removal of pain at wounded part, hemostasis, and prevention of infectious diseases, and can leave scars or the like as little as possible by positive medical treatment to be applied from the start of operation up to cure, with introduction of molecular biochemistry which has not been established in the field of surgery cures.

The above objects can be achieved by the wound-curative water of the present invention, comprising silver-ionic water of pH 5 or less, silver ion concentration of 0.5 to 10 ppm and electric conductance of 250 to 2,000 $\mu$Ʊ $/cm^3$.

In a preferred embodiment of the 5th use of the present invention, the wound-curative water of the present invention may contain activated oxygen in a dissolved oxygen amount of 12 to 30 ppm.

The present invention has introduced the theory of bioinorganic chemistry to have established a remedial method entirely different from the various remedial methods having been hitherto employed in the process of

curing or recovering tissue wounded by injuries or surgeries. In the cure process which begins with receipt of a wound and end with completion of cure, various factors are complicatedly entangled and correlated to give composite effect, and it may be difficult to wholly explain the action of the wound-curative water of the present invention, which, however, can be summarized as follows: (1) Removal of the source of pain by equilibrating the potential difference between the inside and outside of a cell membrane suffering damage and considered to be the source from which a stimulative impulse, called the pain, is emitted, with compulsory introduction of a given electrochemical potential to lower the action potential and at the same time to inactivate the function of the wounded cell membrane; (2) hemostasis by causing unbalance between prostacycline and thromboxanthine by the peroxidation of lipids in the internal walls of broken blood vessels with use of an active enzyme to allow a blood coagulation mechanism to work; (3) prevention of infectious diseases by inclining the environment of wounded part to the acidic side to exterminate the inflammatory nests, and on the other hand by inactivating the function of cell membranes of infectious bacterial bodies by the catalytic function of silver ions; and (4) promotion of cure by inactivating the

function of the cell membranes of wounded cells by the catalytic function of silver ions, cleaning away to remove also the wounded cells together with dead cells, and achieving regeneration with sound and normal cell groups only. The wound-curative water of the present invention can be very effective at the silver ion concentration of 0.5 to 10 ppm which is far lower than 50 ppm, the safty degree of $LD_{50}/ED_{50}$ in human living bodies for silver-ionic water, and the application of the wound-curative water of the present invention can be said to be perfectly of no danger to life.

The wound-curative water of the present invention has the pH value of 5 or less, more preferably pH 4 or less, and most preferably pH 3 or less. The pH value of the present wound-curative water is made to be 5 or less like this to prevent the action of silver ions being lowered because of the adsorption of silver ions to proteins, and at the same time to incline the wounded part toward the acidic side to melt lysosome, thereby promoting the absorptive cleaning of wounded cells. The silver ion concentration may be 0.5 to 10 ppm, more preferably 1 to 7 ppm, and most preferably 3 to 6 ppm. Under the pH of 0.5 to 10 ppm like this, which is far lower than 50 ppm, the safty degree of $LD_{50}/ED_{50}$, silver ions can act only on the wounded cells and pathogenic bacterial bodies at the

wounded part to cure the wounded part without damaging normal cells. The wound-curative water of the present invention further has the electrical conductance of 250 to 2,000 $\mu U/cm^3$, more preferably 1,000 $\mu U/cm^3$ or more, and most preferably 1,500 $\mu U/cm^3$. The electrical conductance is made to be 250 to 2,000 $\mu U/cm^3$ like this to make good the permeability of silver ions to cells, thereby making it possible to allow silver ions to sufficiently exhibit their action, and inactivating and demolishing the wounded cell membranes, the source of pain, to enhance the effect of removing the pain. In addition, the wound-curative water of the present invention has the dissolved oxygen amount of 12 to 30 ppm. This is effective for enhancing hemostatic action and promoting curative action of the wound-curative water of the present invention.

The production of the wound-curative water of the present invention can be carried out without formation of any silver colloids by using the above apparatus as shown in Fig. 1.

The 5th use of the present invention will be described below in greater detail based on working embodiments, but, for convenience sake of explanation, the description will be made separately for each category.

The wounds caused by injuries or surgeries may give pain to human bodies as a matter of course. The pain due

to the wounds is considered to be excitation of cell membranes, which change generally from the state of rest to the state of activity, and actually it is an abrupt electrical change, in other words, generation of action potential, in cell membranes. More specifically, once a shock is given to a nerve, acetyl choline is emitted from vescicles containing acetyl choline present at presynapse membrane to the gap between synapses to combine with a specific receptor present at postsynapse membrane. Then, the permeability of postcynaps greatly changes. Namely, both the ion conductance of $Na^+$ and that of $K^+$ increase, so that a large quantity of $Na^+$ enters a cell from outside and, on the contrary, a little quantity of $K^+$ in the cell flows out to the outside. This is called depolarization due to inflow of $Na^+$, wherein the change of the potential of postcynaps membrane induces action potential to resting nerve fiber membrane adjacent thereto. This spreads to adjacent resting membranes in succession to repeat inflow of $Na^+$ and outflow of $K^+$, whereby the excitation and transmission take place. In general, while the excitation continues, the receptor potential is also maintained and impulses repeatedly generate. However, the present inventors have paid attention to the mechanism in which the generation of excitation in the postsynapse is repressed on the contrary by the action of reppressible

transmitters. This mechanism is such that the permeability of inions to $Cl^-$ temporarily increases to approach the equilibrium potential of $Cl^-$ by the action of the transmitter, and further overlaps with the short-circuit effect that lowers the electrical resistance of the membrane because of the increase of the permeability, whereby the action potential decreases and finally the amount of release of excitable transmitters is lowered. The present inventors have also paid attention to the fact that the emission source of the stimulation impulse is in the wounded cell membranes. From these viewpoints, they considered that certain electrochemical potential may be given to $Cl^-$ and $Na^+$ participating in the cells damaged by injuries or surgeries and may be forced to flow into the cells, whereby the potential difference may be equilibrated, the action potential may be decreased, and thus the emission of the impulse of the pain may be stopped. They have further considered that the pain may be perfectly removed by inactivating the function of the cell membranes of the wounded cells. Based on such thoughts and as a result of intensive studies, the application of the silver-ionic water of pH 5 or less, silver ion concentration of 1 to 7 ppm and electrical conductance of 250 to 2,000 $\mu\mho/cm_3$ lowered the action potential of the membranes, and thus they have succeeded

in temporarily stop the pain. Further, the silver ions flowed into the wounded cells changed unsaturated aliphatic acids in phospholipids constituting the cell membranes into ethylene by the catalytic action inherent only in the silver ions for 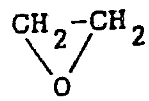, to inactivate the function of membranes and demolish the emission source of the impulse of the pain, and thus they have succeeded in perfectly stop the pain.

Next, injuries or surgeries may result in the break of blood vessels and bleeding, as a matter of course. The bleeding from blood capillaries may temporarily stop soon or later with formation of clots, as platelets stick with time lapse to collagen exposed by the cut of blood vessels, but may not stop during operation to bring about an obstruction factor for the operation. Also, after the operation, it sometimes may happen that hemostasis at the wounded part is not perfectly effected, whereby bloodtumors are formed and dead cavities remain at the wounded part to delay the recovery. The physiological mechanism of hemostasis is such that the prostacycline which is a coagulation inhibitory substance constantly released from blood cell walls is stopped being released when blood endothelial cells are damaged, and platelets stick to the wounded part to make vigorous the synthesis

of thromboxanthine, whereby the formation of thrombi is accelerated. In order to artificially and rapidly cause this, the present inventors, as a result of intensive studies, have found the fact that there occurrs unbalance between prostacycline and thromboxanthine in blood vessel obturation in primaturity retinopathy, and, on the basis thereof, have noted that the mechanism of the formation of thrombi originates from the methabolism of prostaglandin, which is accompanied with the peroxidation reaction. Then, the silver-ionic water obtained by increasing the concentration of the dissolved oxygen in the silver-ionic water to about 12 to 30 ppm which was nearly twice that of ordinary water, and at the same time activated to increase the oxidation power by about 1,500 times, was sprayed on the wounded part. As a result, the bleeding was stopped as there was presumably caused the blood coagulating mechanism such that peroxidation of lipids in the broken blood vessels proceeded, prostacycline was artificially inhibited, thromboxanthine was released, complexes of prothrombin $^{-}$Ca were adhered to the phospholipid proteins released from platelets, the prothrombin hydrolyzed by factor activators Xa was further transformed into thrombin, and separated from phospholipids. Thus, bleeding was immediately stopped in the case of injuries, and, in the case of surgeries, no exudation of blood

occurred at all to bring about a great change in the surgery environment.

The sound and normal skin of living bodies not only plays a role as a mechanical barrier to pathogenic bodies, but also plays a role as a chemical barrier by virtue of spermine, lysozyme, etc. contained in secretions from the skin. However, the cells damaged by injuries or surgeries themselves may lose the viability originally maintained in tissue, so that it may often occur that the infection resistance is weakened and inframation due to bacteria, fungi, viruses or protosoa is caused. Until the chemotherapeutic agents, antibiotics, antibacterial agents were developed, acute inflammation was principally cured by local static cooling, wet compressing, or depression drainage by dissection or the like, and chronic inflammation was principally cured by dissection drainage, sweeping-away of inflammatory internal buds, or ablation of festered walls. Nowadays, however, administration of broad spectrum antibiotics in a large quantity has borne its fruit in the prevention of infection. However, this has instead caused fixation of resistance bacteria to organs, leading it to the so-called opportunistic infectious diseases. This is an intractable disease which has been caused because the bactericidal or bacteriostatic effect could not have been expected on all the bacteria as

the chemical drugs have pursued effect corresponding to the respective bacteria and have been prepared and administered within the scope of the safty degree of $LD_{50}/ED_{50}$. Now, the present inventors has considered in a series of researches that the environment at the wounded part may be made to have the pH of 5 or less, in other words, inclined to the acidic side, thereby melting lysosome in the granules, leukocytes or wounded cells which are sensible to the lowering of the pH, and releasing collagenase and so forth contained therein to exterminate inflammatory nests, and, on the other hand, since all the infectious bacterial bodies ranging from viruses to protosoa are common to each other in that they have cell membrane, the bacteria may be killed by inactivating the function of the cell membranes. Then, silver-ionic water of pH 5 or less, silver ion concentration of 0.5 to 10 ppm, electric conductance of 250 to 2,000 $\mu U/cm^3$ was sprayed on a wounded surface. As a result, together with the effect of melting collagenase, there was seen the effect that formation of ethylene of 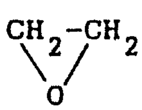 took place in the cis structure of $-CH_2-$ of an aliphatic group which is hydrophobic part of phospholipids constituting the cell membranes, by the action of the catalytic action inherent in the silver ions flowed into

bacterial bodies according to the difference in electrochemical potential of silver ions, to cause inactivation of cell membranes, whereby water flowed in from the outside to break and kill the cells. As will be supported by the fact that the safety degree of silver ions to human bodies according to $LD_{50}/ED_{50}$ is 50 ppm and, as compared with this, the wound-curative water of the present invention has the concentration of as low as 0.5 to 10 ppm, the above silver-ionic water gave no damage at all to normal tissue cells.

With regard to the cure of wounds, what is important for promoting the process of cure is to positively remove inhibitory factors for cure to accelerate the progress of cure. Among such inhibitory factors, the bleeding and infectious diseases can be removed in the manner as mentioned above. The present inventors have further paid attention to the participation of damaged cells as an inhibitory factor.

It is presumed that the process of the cure of wounds may not only be complicated depending on the degree of damage but also be a recovery work carried out while dealing with the cells having a weakened resistance to the infection, so that there may be complicatedly mingled with various processes such that the cells may necrotize on route or the necrotized cells must be separated and

excluded, resulting in the delay of cure. Moreover, although it is an important function that the lukocytes or histiocyte emigrating from blood cell walls may melt or digest dead cells of the wounded tissue, this may on the other hand produce a state of edema to delay the cure. Then, the present inventors attempted to remove the cells wounded to have a weakened resistance, by inactivating the function of the cell membranes thereof, thereby achieving the regeneration only with sound and normal cell groups, which is a remedial method that has not been available.

Silver-ionic water of pH 5 or less, silver ion concentration of 0.5 to 10 ppm, electrical conductance of 250 to 2,000 $\mu\mho/cm^3$ and a dissolved oxygen amount of 12 to 30 ppm was sprayed on a wound with use of an atomizer to proceed with an operation. As a result, even with the ion concentration of 0.5 to 10 ppm which is far lower than 50 ppm which is the safety degree of $LD_{50}/ED_{50}$ for human living bodies, the wounded cells, having a weak resistance, was inactivated by the catalytic function of silver ions to lose its function of controlling inflow of substances at the cell membranes, and broken by inflow of water from the outside of the cells. Further, lysosomes were broken under the state of pH 5 or less to release collagenase to melt proteins. Also, at the same time, in respect of pathogenic bodies that invaded, the function of

cell membranes was also inactivated and broken to give death of them by the action of the silver ions as mentioned above. These protein high molecular elements were melted and absorbed in the tissue, and, on the wounded surface, not only the mechanism of reactivation started only with sound and normal cells immediately after suture, but also the scars after cure were restricted to a minimum, and became almost quiet with lapse of time.

[Examples of the 5th use]

The present invention will be described below in still greater detail by Examples.

Example 5-1

Symptom: Laceration from the root of the left thumb finger to the wrist with a razor-like edged tool. The wounded part had a depth of 5 mm and length of 3.5 cm with plenty bleeding so that the wrist was in the state that it was tighed with cloth for emergency hemostasis.

Treatment:

With silver-ionic water of pH 3, silver ion concentration of 4 ppm, electrical conductance of 800 $\mu$U $/cm^3$ and a dissolved oxygen amount of 20 ppm, wounded surface was washed and the wound was taped.

Cure effect:

The pain stopped in about 3 minutes, and the bleeding perfectly stopped a little later. Tape was

released after 2 days, with wounded part perfectly accreted.

Example 5-2

Symptom: Leceration by acropressure at the second joint part of the right forefinger. The pain lasted even one week after surgery suture by an arthopaedist, with loss of sleep of the patient.

Treatment:

Dipped for 5 minutes in silver-ionic water of pH 3, silver ion concentration of 4 ppm, electrical conductance of 1,500 μU/cm$^3$ and a dissolved oxygen amount of 20 ppm.

Cure effect:

The pain was eased 5 minutes after the treatment, with no feel of pain in 10 minutes.

Example 5-3

Symptom: Blood tumor in the head. Coronale was dislocated to carry out an operation to deliver the tumor.

Treatment:

Silver-ionic water of pH 3.5, silver ion concentration of 4 ppm, electrical conductance of 1,500 μU /cm$^3$ and a dissolved oxygen amount of 20 ppm was sprayed with a sprayer at intervals of 15 minutes for 1 hour and 30 minutes of operation time.

Effect:

The bleeding from minute blood vessels stopped in 1

minute after spraying, and the spraying at intervals of 15 minutes effected no bleeding seen by the end of the operation, with very good progress after operation.

Example 5-4

Symptom: Because of the right femur fracture, insection was carried out. Sutured after insertion of a center metal.

Treatment:

Silver-ionic water of pH 3, silver ion concentration of 4 ppm, electrical conductance of 1,500 $\mu\mho/cm^3$ and a dissolved oxygen amount of 20 ppm was sprayed before operation on the part to be treated and then wiped, and hands of surgeons and assistants were sterilized by dipping them in the same silver-ionic water for 3 minutes. The same silver-ionic water was sprayed at intervals of 10 minutes during 45 minutes of the operation time. After finish of the operation, a wet compress using the same silver-ionic water was applied for 12 hours.

Effect:

Even after anesthetic effect was lost, the patient complained of no pain. Also, no bleeding from minute blood vessel and no exudation from blood vessel walls were seen during the operation, and the cure was so speedy that a white regenerated layer considered to be comprised of collagen was formed with a width of about 5 mm after 24

hours. Stitches were taken away after 2 days, and thereafter a wet compress was also applied successively, whereby no contractile scar was seen at all.

Example 5-5

Symptom:

Abrasion at the left elbow, extending toward upper brachium. Some abrasion extended from a corneal layer of epidermis to a basal layer. Wounded part had a width of about 4 cm and length of about 20 cm.

Treatment:

Washed with silver-ionic water of pH 2.5, silver ion concentration of 4 ppm, electrical conductance of 1,600 $\mu$U $/cm^3$ and a dissolved oxygen amount of 20 ppm, and thereafter a wet compress using the same silver-ionic water was applied.

Effect:

The pain stopped in about 3 minutes, and a membranous surface layer appeared after 24 hours. A wet compress was successively applied, and, after further 24 hours, a thin leather-like scarred skin was formed, which was not hardened and was peeled after 3 days without any scars and without any difficulty in stretching the arm.

As described in the foregoing, the present invention is the wound-curative water comprising silver-ionic water of pH 5 or less, silver ion concentration of 0.5 to 10

ppm, and electrical conductance of 250 to 2,000 μU/$cm^3$, and therefore can be favorably used for cure of wounds and for surgeries, that can facilitate removal of pain at wounded part caused by injuries or surgeries; hemostasis, prevention of infectious diseases, and promotion of the recovery of tissue.

A 6th use of the silver-ionic water of the present invention is concerned with a disinfectant water that can disinfect, in surgeries, hands and arms of surgeons and assistants, operation field of patients, and tools and equipments, and can prevent exogeneous or endogeneous infectious diseases being caused after the operation.

In general, disinfectants refer to chemical substances that can kill pathogenic microbes to prevent the infection by pathogenic bodies. The disinfectants, different from chemotherapeutic agents, can be applied not only on apparatus, tools, cloth, bedcloths, interiors, food, filth, extrement, etc., but also on living body tissue such as sound skin, mucosa, wounded surfaces, pustulated surfaces and surgery wounds.

The kind of pathogenic microbes to be targeted by disinfectants may include wide range ones classified into procalyotes such as bacteria and Cyanophyta, and

eucaryotes such as fungi, Myxomycetes, algae, and protozoa. The type of bacteria, for example, may also include a group of gram-positive coccus, a group of gram-negative coccus, a group of gram-positive Bacillus, a group of gram-negative Bacillus, strict anaerobe, Mycobacterium, Spirochaeta, Chlamydia, Rickettsia, etc.

However, these are common to each other in that all of them have cell membranes and cell walls. Now, the disinfectants having been hitherto available can be grouped into the chemical agents synthesized aiming at the following four functions or mechanisms. Namely, i) inhibition of cell walls, ii) inhibition of cell membranes, iii) inhibition of synthesis of proteins, or iv) inhibition of synthesis of nucleic acids and DNA. Like this, the presently available disinfectants depend on the inhibitory effect against living bodies. However, for example, the composition of the cell membranes commonly owned by the pathogenic microbes is common not only to that of these pathogenic microbes but also to that of cell membranes of animals. Thus, it is an important problem that the presently available disinfectants, different from antibiotics or chemotherapeutic agents, are non-selective and can give disorders also to animal bacteria.

At present, phenols, aldehydes, alcohols, acids, halides, dyes, bimetallic compounds, surface active

agents, etc. are used as disinfectants, which respectively may have advantages and disadvantages, may have necessarily limitation in the chemicals to be used or the concentration thereof depending on targets, and can not cope with pathogenic microbes in all types of infection sources.

Accordingly, to arrange the conditions generally required for disinfectants, there may be mentioned (i) strong disinfectant effect, (ii) broad antibacterial spectrum, (iii) speedy achievement of disinfectant effect, (iv) strong permeability, (v) effectiveness even under the conditions of blood, sputum, urine, etc. in which proteins are co-present, (vi) no adverse effect on targets of disinfection, (vii) no problem in storage and transportation as being chemically stable, (viii) inexpensiveness, and so forth, and development of a disinfectant meeting these requirements has been sought after.

A first problem that may arise as a result when no sufficient disinfection or sterilization has been effected by such a disinfectant is concerned with infectious diseases that may caused after surgical operations. This problem is important in view of the fact that in the patients who have had surgical operations, the rate of suffering infectious diseases is such that it is 8 % in

220 cases for younger than 40 year old patients; 14 % in 301 cases for 50 to 59 year old patients; and 25 % in 122 cases for 70 or more year old patients, as reported in certain data available in the last one year (data from Shiro Hayashi, "Basic Surgery", Asakura Shoten, p.167, "Selection of Antibacterial Agents").

Before operation, disinfection of skin is carried out for patients as a matter of course, but, on the other hand, bacterial floras are abundantly present also in mucosal portions such as mouth cavity and intestinum rectum. Moreover, no sufficient effect of disinfectants can be obtained in the diseased part made wet and covered with a mucosal layer because of the flowing-out of secretion, and such part has so weak resistance that any strong disinfectant as used for the skin can not be used.

In operations that must absolutely prevent infection, for example, the intracarnial operation, the operation where synthetic materials are used for artificial valve replacement, artificial blood vessel, artificial condylus replacement and so forth, perfectness is required for cleaning of operation environment and also for disinfection of operation field. Thus, it is very difficult to select disinfectants depending on the part or purpose of operation, and the state of things in the present stage is such that there can not be seen any

agents and preventive methods satisfying these requirements.

Accordingly, a still further object of the present invention is to provide a novel disinfectant water. The present invention also aims at providing a disinfectant water that can disinfect, in surgeries, hands and arms of surgeons and assistants, operation field of patients, and tools and equipments, and can prevent exogeneous or endogeneous infectious diseases after the operation. The present invention further aims at providing a disinfectant water taking into consideration the protective system for skin and mucosa. Still further, the present invention aims at providing a disinfectant water that can have broad antibacterial spectrum, can have speedy pharmaceutical effect, which can yet be maintained for a long period of time, and also can be non-stimulant, atoxic, odorless and colorless.

The above objects can be achieved by the disinfectant water of the present invention, comprising silver-ionic water of pH 5 or less and silver ion concentration of 0.5 to 10 ppm.

In a preferred embodiment of the present invention, the disinfectant water may have electrical conductance of 350 to 2,000 $\mu\mho/cm^3$.

The 6th use of the silver-ionic water of the present

invention is based on a mechanism entirely different from that of the conventional disinfectants, and the same mechanism as that for the 4th use of the invention, the therapeutic water for pathogenobacterial skin diseases and external cavity mucosal diseases. Explanation for it is not repeated here, accordingly.

The specific action of the 6th use of the present invention will be described below.

All the cells, ranging from animal cells to pathogenic microbes such as bacteria, fungi, viruses, protozoans, are enveloped with cell membranes, having an inseparable relationship between these membranes and cell bodies, and life can not exist unless membranes exist, which membranes form boundaries to prevent outflow of biotic substances and inflow of harmful substances.

Having analyzed the substance constitution of cells, there are contained 25 to 60 % of polar lipids to construct the fundamental structure with lipid double layers and membrane proteins. The phospholipids contained therein are rich in readily oxidizable polyvalent unsaturated aliphatic acids. In the polar lipid molecules, hydrophilic part is in direct contact with an aqueous phase and has electric resistance of as low as $10^2$ to $10^5$ $\Omega/cm^3$. On the other hand, hydrophobic part contains a large quantity of $-CH_2-$ such as aliphatic

acids, which are arranged in parallel to each other by hydrophobic bond to form double structure.

Here, inactivation of cells, when viewing the manner in which cell membranes themselves are inactivated even in a natural environment as cell membranes actually participate therein, radicals of the polyvalent unsaturated aliphatic acids are formed when peripheral ion type substances or OH groups are formed in the vicinity of membranes, and combine with biradical oxygen to form aliphatic acid peroxyradicals to successively cause hydrolysis, and finally the membranes are broken. In order to artificially cause this mechanism to bring about to inactivate target bacteria, a catalyst capable of causing these chemical reactions is required, and there can be found nothing other than silver as the substance having such functions. The catalytic action of the silver, that can form $C_2H_4O$ as a partially oxidized product in the oxidation reaction of ethylene, is a selective action that no other metallic ion can achieve. Accordingly, the disinfectant water of the present invention contain the silver having the above action as silver ions in concentration of 0.5 to 10 ppm, more preferably 2 to 7 ppm, most preferably 4 to 6 ppm. Thus, the silver ions can act on all the bacterial bodies targeted by the disinfectant water of the invention as

explained later, at the concentration of 0.5 to 10 ppm which is far lower than 50 ppm which is the safety degree of $LD_{50}/ED_{50}$, and on the other hand has no problem for the mucosal portions which have weak resistance as compared with the skin, without any adverse effect against normal tissue cells.

The disinfectant water of the present invention may have the pH value of pH 5 or less, more preferably pH 4 or less, and most preferably pH 3 or less. Making the disinfectant water to have the pH value of 5 or less as above, adsorption of silver ions on proteins can be prevented, a lowering of the catalytic action of silver ions can be prevented even under the conditions of co-presence of proteins, and it is made possible for silver ions to freely permeate the cell membranes of target bacterial bodies.

The disinfectant water of the present invention may further have electrical conductance of 350 to 2,000 $\mu U/cm^3$, more preferably 1,000 $\mu U/cm^3$ or more. Making the disinfectant water to have the electrical conductance of 350 to 2,000 $\mu U/cm^3$, the cell permeability of silver becomes better, and it is made possible for the action of silver ions to be sufficiently exhibited.

The production of the disinfectant water of the present invention can be favorably carried out by using

the aforesaid apparatus shown in Fig. 1.

The disinfectant water according to the 6th use of the silver-ionic water of the present invention will be described below in greater detail based on working embodiments.

The present inventors have first surveyed bacteria habitually present in human bodies and examined the effect the disinfectant water of the present invention may have on these. Certain microbes are fixed on the portions being in direct or indirect contact with outside, such as skin, oral cavity, alimentary canals and vulva. These include permanent bacterial floras and temporary bacterial floras. The former may little serve as infectious bacteria for humans, but some of the bacteria may excessively propagate themselves because of administration of antibiotics to cause infection, the so-called bacterial alternation diseases or opportunistic infectious diseases, in the patients having weak resistance. The latter may comprise bacterial species temporarily propagated, which invaded from a bacterial nest environment or from other intracorporea, to very frequently cause infectious diseases. Now, the disinfectant water of the present invention revealed to be able to inactivate all the habitually present bacteria including gram-positive bacteria such as $\alpha,\gamma$-Streptocuccus, $\beta$-Streptococcus,

Diplocuccus pneumoniae, enterocuccus, yellow Staphylococcus, coagulase-negative Staphylococcus, Peptococcus and Peptostreptococcus; gram-positive bacilli such as Corynebacterium diphteriae, Bacillus, Propionibacterium, Lactobacillus lactis, Corynebacterium and Actinomyces; gram-negative baccilli such as Haemophilus, Escherichia coli, Pseudomonas aeruginosa, Bacteroides, B. fragilis, Fusobacterium and anerobic vibrio; Clostridium; acid-fast bacterial group; mycoplasmas; Spirohaeta; etc.

Next, examined was the effect of the disinfectant water of the present invention, against bacterial floras in an environment. The bacterial floras in an environment may include bacterial floras respectively present in the ground, in the water and in the air, and it may be not so possible that the infection may occur in the modern operation rooms. However, bacterial floras in hostpitals may live at sinks, cocks on waterworks, filth-treating equipments, and other moist places, may be the bacteria resistant to various agents, and may cause the nosocomial infection by enterobacteria such as Pseudomonas aeruginosa, Pseudomonas cepacia and Achromobacter, and Staphylococcus. The disinfectant water of the present invention had a dramatical effect presumably because the bacteriostatic or bactericidal mechanism is entirely

different from that of the conventional agents.

The disinfectant water of the present invention was further examined for the effect against pathogenic bodies of microbes such as fungi, viruses and protosoa, to reveal that it can effectively act on all the pathogenic bodies to inactivate them, and that it can have broad antimocrpbial spectrum and can achieve a strong disinfectant effect and a speedy disinfectant effect.

Subsequently, the present inventors have made studies on the permeability of the disinfectant water of the present invention.

The protective system against the infection of sound skin by bacteria may include a keratin layer, a sebum membrane on its surface, and an epidermal protective membrane beneath the keratin layer, Thickness of the keratin layer may vary according to the portion of a body, and 10 to 20 µm at the palm of the hand or the planta pedis, but as thin as 5 µm at the abdominal wall. The sebum membrane is constituted of secretion from serum gland and a product of epitherium, and has a principal component of a low molecular aliphatic acid. The surface of the skin has the pH of 5 to 6 in average, which pH may return to normal value after a certain time by virtue of the secretion of sebum even when the skin has been exposed to weak acid or weak alkali. Such properties of the sebum

membrane can prevent the propagation of bacteria on the surface of the epidermis even if the skin is inadvertently parasitized by the bacteria. The epidermal protective membrane beneath the keratin layer can prevent water electrolytes or the like entering it from the outside. This epidermal protective membrane appears to have considerably strong protective system, but, at the same time therewith, has an infinit number of dermal appendages penetrating the epidermis and dermis, in other words, fine lumina such as ormental burse, sebums and sweat glands, which may have an important meaning with respect to the disinfection of the skin in the operation field of hand fingers and arms in an operation. A great number of bacteria is present at the skin, and they can be grouped into the bacterial floras adhering on the skin surface and the bacterial floras concealed in ormental burse, sebums and sweat glands. The conventional disinfection has principally aimed at decreasing the bacterial floras adhered on the skin surface, and has been powerless to the latter bacterial floras.

However, as a result of an experiment carried out against the bacterial floras in ormental burse, sebums and sweat glands of surgeons and assistants with use of the disinfectant water of the present invention, it was established that the silver-ionic water of the present

invention evenly permeated the skin from the openings of sebaceous glands into terminal secretory portions owing to the inclination of electrochemical potential, and completely exterminated the bacteria lived in the inside, whereby most of the bacteria released along with the perspiration in an operation requiring an operation time extending over a long time were inactivated to give a sterilized state, and thus the disinfectant water of the invention had a high permeability.

The present inventors have further made studies concerning the disinfection of mucosal portions, which has been another problem in the conventional disinfectants.

In an intraoral operation or an operation at a portion relating to alimentary canals such as intestinum rectum, questioned are the bacterial floras present in the mucosa placed in its operation field. In such mucosa, bacterial floras are abundantly present, but the strong disinfectants conventionally used for the skin disinfection can not be used at the mucosal portions having weak resistance. Moreover, such portions are in such a state that the secretion continually flows out to make moist them and they are covered with mucosal layers to make it difficult to have the effect of disinfectants. However, the disinfectant water of the present invention, which has silver ion concentration of as very low as 0.5

to 10 ppm as compared with the fact that the safety degree of $LD_{50}/ED_{50}$ is 50 ppm as mentioned above, had a high permeability to make it possible to completely inactivate the bacterial floras present beneath the mucosal layer, without any adverse effect such as stimulation and cell injury in the mucosal portions.

Accordingly, it was found that the disinfectant water of the present invention can be used with very excellent bacteriostatic or sterilizing effect on any targets including not only not only apparatus, tools, cloth, bedcloths, interiors, food, filth, extrement, etc., but also living body tissue such as sound skin, mucosa, wounded surfaces, functional wounds and surgery wounds.

[Examples of the 6th use]

Example 6-1

Before starting an operation, hands were cleaned according to the following respective manners, and the number of bacteria led out along with the perspiration was measured after the operation was finished.

i) Comparative example: Hands were washed with use of invert soap, thereafter dried with a drier, and gloved.

ii) Example: Right and left hands were thoroughly rubbed each other, thereafter dipped in silver-ionic water, thereafter dried with a drier, and gloved.

Also, the right hand was dipped in silver-ionic

water of pH 4, and the left hand was dipped in silver-ionic water of pH 3. There were prepared three types of silver-ionic water having the silver-ion concentration of 0.5 ppm, 2 ppm and 4 ppm, respectively.

Results thus obtained are shown in Table 11 to Table 17. It is seen from these results that there can be obtained more remarkable disinfectant effect by dipping hands in the silver-ionic water than in the case the invert soap was used.

Table 11 (Invert soap)

| Operator | Before hand-washing (number/ml) | | | After hand-washing (number/ml) | | | 3 hr. after operation (number/ml) | | |
|---|---|---|---|---|---|---|---|---|---|
| | General bacteria | E. coli | Staphylococcus | General bacteria | E. coli | Staphylococcus | General bacteria | E. coli | Staphylococcus |
| A | 1,800 | 0 | - | 20 | 0 | - | 1,500 | 0 | - |
| B | 4,800 | 0 | - | 45 | 0 | - | 750 | 1 | - |
| C | 150 | 5 | + | 7 | 0 | - | 2,600 | 3 | + |
| D | 3,200 | 0 | - | 35 | 0 | - | 2,100 | 0 | - |
| E | 500 | 20 | + | 10 | 0 | - | 1,400 | 1 | - |

Table 12 (Silver-ionic water; Ag ion conc.: 0.5 ppm; pH 4) (Right hand)

| Operator | Before hand-washing (number/ml) | | | After dipping for 2 min. (number/ml) | | | 3 hr. after operation (number/ml) | | |
|---|---|---|---|---|---|---|---|---|---|
| | General bacteria | E. coli | Staphylococcus | General bacteria | E. coli | Staphylococcus | General bacteria | E. coli | Staphylococcus |
| A | 1,420 | 0 | - | 1 | 0 | - | 12 | 0 | - |
| B | 3,120 | 3 | - | 2 | 0 | - | 30 | 0 | - |
| C | 120 | 1 | - | 0 | 0 | - | 0 | 0 | - |
| D | 150 | 0 | - | 0 | 0 | - | 0 | 0 | - |
| E | 1,100 | 7 | + | 2 | 0 | - | 21 | 0 | - |

Table 13 (Silver-ionic water; Ag ion conc.: 0.5 ppm; pH 3) (Left hand)

| Operator | Before hand-washing (number/ml) | | | After dipping for 2 min. (number/ml) | | | 3 hr. after operation (number/ml) | | |
|---|---|---|---|---|---|---|---|---|---|
| | General bacteria | E. coli | Staphylococcus | General bacteria | E. coli | Staphylococcus | General bacteria | E. coli | Staphylococcus |
| A | 1,360 | 1 | - | 0 | 0 | - | 4 | 0 | - |
| B | 2,891 | 4 | + | 1 | 0 | - | 7 | 0 | - |
| C | 124 | 1 | - | 0 | 0 | - | 0 | 0 | - |
| D | 171 | 1 | - | 0 | 0 | - | 0 | 0 | - |
| E | 1,100 | 10 | + | 2 | 0 | - | 5 | 0 | - |

Table 14 (Silver-ionic water; Ag ion conc.: 2 ppm; pH 4) (Right hand)

| Operator | Before hand-washing (number/ml) | | | After dipping for 2 min. (number/ml) | | | 3 hr. after operation (number/ml) | | |
|---|---|---|---|---|---|---|---|---|---|
| | General bacteria | E. coli | Staphylococcus | General bacteria | E. coli | Staphylococcus | General bacteria | E. coli | Staphylococcus |
| A | 750 | 1 | - | 0 | 0 | - | 0 | 0 | - |
| B | 1,010 | 2 | - | 0 | 0 | - | 3 | 0 | - |
| C | 610 | 1 | - | 0 | 0 | - | 0 | 0 | - |
| D | 2,200 | 6 | + | 0 | 0 | - | 3 | 0 | - |
| E | 570 | 0 | - | 0 | 0 | - | 1 | 0 | - |

Table 15 (Silver-ionic water; Ag ion conc.: 2 ppm; pH 3) (Left hand)

| Operator | Before hand-washing (number/ml) | | | After dipping for 2 min. (number/ml) | | | 3 hr. after operation (number/ml) | | |
|---|---|---|---|---|---|---|---|---|---|
| | General bacteria | E. coli | Staphylococcus | General bacteria | E. coli | Staphylococcus | General bacteria | E. coli | Staphylococcus |
| A | 690 | 2 | - | 0 | 0 | - | 0 | 0 | - |
| B | 1,204 | 1 | - | 0 | 0 | - | 0 | 0 | - |
| C | 610 | 0 | - | 0 | 0 | - | 0 | 0 | - |
| D | 2,200 | 5 | + | 0 | 0 | - | 0 | 0 | - |
| E | 625 | 0 | - | 0 | 0 | - | 1 | 0 | - |

Table 16 (Silver-ionic water; Ag ion conc.: 4 ppm; pH 4) (Right hand)

| Operator | Before hand-washing (number/ml) | | | After dipping for 2 min. (number/ml) | | | 3 hr. after operation (number/ml) | | |
|---|---|---|---|---|---|---|---|---|---|
| | General bacteria | E. coli | Staphylococcus | General bacteria | E. coli | Staphylococcus | General bacteria | E. coli | Staphylococcus |
| A | 825 | 1 | - | 0 | 0 | - | 0 | 0 | - |
| B | 710 | 0 | - | 0 | 0 | - | 0 | 0 | - |
| C | 150 | 2 | + | 0 | 0 | - | 1 | 0 | - |
| D | 2,030 | 0 | - | 0 | 0 | - | 0 | 0 | - |
| E | 527 | 0 | - | 0 | 0 | - | 0 | 0 | - |

Table 17 (Silver-ionic water; Ag ion conc.: 4 ppm; pH 3) (Left hand)

| Operator | Before hand-washing (number/ml) | | | After dipping for 2 min. (number/ml) | | | 3 hr. after operation (number/ml) | | |
|---|---|---|---|---|---|---|---|---|---|
| | General bacteria | E. coli | Staphylococcus | General bacteria | E. coli | Staphylococcus | General bacteria | E. coli | Staphylococcus |
| A | 802 | 1 | – | 0 | 0 | – | 0 | 0 | – |
| B | 1,010 | 0 | – | 0 | 0 | – | 0 | 0 | – |
| C | 210 | 2 | + | 0 | 0 | – | 0 | 0 | – |
| D | 2,100 | 0 | – | 0 | 0 | – | 0 | 0 | – |
| E | 570 | 1 | – | 0 | 0 | – | 0 | 0 | – |

Example 6-2

To 1 g of dejection, about 5 ml of a sterilized physiological saline solution was added and the mixture was thoroughly mixed, to which silver-ionic water of different types was added, and the mixture was cultured for a prescribed time. Thereafter, the number of Enterococcus contained in it was measured.

Prepared were 4 types of silver-ionic water of pH 5, pH 4, pH 3 and pH of less than 3, having the silver ion concentration of 1 ppm and 4 ppm, respectively.

Results obtained are shown in Table 18 and Table 19. Thus, employment of the silver-ionic water made it possible to greatly decrease bacteria in the dejection and favorably keep a sanitary state.

Table 18

(Test for effect of silver-ionic water, using dejection)

(1 ppm)

| | Control | pH 5 | pH 4 | pH 3 | < pH 3 |
|---|---|---|---|---|---|
| A | $3.4x10^4$ | $8.0x10^2$ | $2.4x10^3$ | $2.1x10^3$ | $5.1x10^2$ |
| B | $5.1x10^6$ | $2.2x10^4$ | $9.1x10^3$ | $9.2x10^2$ | $7.1x10^2$ |
| C | $4.3x10^5$ | $6.2x10^3$ | $7.3x10^3$ | $1.1x10^3$ | $6.3x10^2$ |
| D | $2.4x10^5$ | $9.1x10^3$ | $1.1x10^3$ | $3.4x10^2$ | $1.1x10^2$ |
| E | $3.1x10^6$ | $8.2x10^3$ | $2.1x10^3$ | $6.1x10^2$ | $2.3x10^2$ |

Table 19

(Test for effect of silver-ionic water, using dejection)

(4 ppm)

| | pH 5 | pH 4 | pH 3 | < pH 3 |
|---|---|---|---|---|
| A | $6.1 x 10^2$ | $4.1 x 10^2$ | 41 | 0 |
| B | $3.2 x 10^3$ | $2.1 x 10^2$ | 21 | 0 |
| C | $7.8 x 10^2$ | $5.3 x 10^2$ | 5 | 0 |
| D | $5.6 x 10^3$ | $2.3 x 10^2$ | 7 | 0 |
| E | $6.2 x 10^2$ | $3.5 x 10^2$ | 0 | 0 |

As described in the foregoing, the present invention

is the disinfectant water characterized by comprising silver-ionic water of pH 5 or less and silver ion concentration of 0.5 to 10 ppm, therefore can be a disinfectant that can have a broad antimicrobial spectrum, can achieve speedy effect as an agent, can maintain the effect for a long tim, can be of no stimulus, odorless and colorless, and can be used for the disinfection of targets of broader range including not only apparatus, tools, cloth, bedcloths, interiors, food, filth, extrement, etc., but also living body tissue such as sound skin, mucosa, wounded surfaces, pustulated surfaces and surgery wounds. Particularly, it can be applied in the disinfection or sterilization of hands and arms of surgeons and assistants and operation field of patients at the time of a surgical operation to prevent exogeneous or endogeneous infectious diseases being caused after the operation.

A 7th use of the silver-ionic water of the present invention is concerned with a therapeutic water for digestive organ mucosal diseases including ulcers such as gastric ulcer and duodenal ulcer in digestive organ mucosa and diarrhea such as bacterial enteritis, and for keratoconjunctivitis caused by the infection with pathogenic microbes such as viruses.

In the diseases in digestive organ mucosa and

keratoconjunctiva of eyes, the mucosal portions of these have very weak resistance, and can not be administered with strong chemical drugs or the like. For these reasons, the therapy of the affected part has been carried out by a negative method in which the autotherapy is relied on while an weakly effective drug is administered, or the ablation of the affected part by a surgery, giving a very large load to the patient.

For example, most of ulcers in digestive organ mucosa such as gastric ulcer and duodenal ulcer are caused by the hypofunction of these gastric organs or by excessive drinking and eating, mental stress, etc. to give hardness on the digestive organs, lose the balance in the various digestive juices, and to produce damage on the mucosal portions. As a therapeutic method therefor, chiefly employed is a method in which an antacid is administered while taking care of giving loads as little as possible to these digestive organs so that the autotherapy may be promoted. When symptoms are progressed, it has been carried out to ablate the ulcerated portion by a surgery.

In the case of the diarrhea caused by the infection with bacteria such as Salmonella in the intestinum rectum and intestinum crassum, it is also impossible to use a strong drug since the mucosa has only a weak resistance to

chemical drugs, and the therapy has been carried out by administering an intestinum regulating agent so that the enteral bacterial floras can recover a normal state. Accordingly, this necessarily requires a long period for the cure.

The epidemic keratitis which is one of the keratoconjunctivitis, which is caused by the infection with a certain kind of adenovirus, is usually cured in 3 to 4 weeks, but kelatoleukoma sometimes takes several weeks to several months, or in a worst case, several years to cure and the therapy has been carried out by using eye drops such as antibiotic eye drops and steroid type eye drops. However, there can be used only drugs having weak bactericidal effect since other functions of eyes may be damaged if any drugs with increased pharmaceutical effect are used, and, on the other hand, there can be developed resistant bacteria that may bring about less effect of the drugs.

The chemotherapeutic agents presently used and having the bacteriostatic or bactericidal effect may sometimes give a shock to a living body to damage the same, many of which may bring about the pharmacological side effect depending on the amount of the drugs and can not necessarily have sufficient selective toxicity to animal cells, so that they can be applied only with

difficulty for the digestive organ mucosa or the keratoconjunctiva of eyes. In particular, application of them for the eye where optic nerves are centered must be further cautious taking account of the adverse effect to these.

Also, the regeneration and recovery of damaged tissue of the portions ulcerated by gastric ulcer or duodenal ulcer have been principally relied on the mechanism relating to the autotherapy, and the administration of antibiotics or the like has been carried out only for the purpose of secondarily preventing the therapy being inhibited by the pathogenic microbial infection. Thus, there has been hitherto found no therapeutic method that can positively promote the regeneration and recovery.

From the viewpoints as mentioned above, as a therapeutic method for the digestive organ mucosal diseases and keratoconjunctivitis of humans, it has been sought after to develop a positive therapeutic method based on new functions and mechanisms, in place of the presently available negative therapeutic method.

Accordingly, a still further object of the present invention is to provide a novel therapeutic water for digestive organ mucosal diseases and keratoconjunctivitis. The resent invention also aims at providing a therapeutic

water for digestive organ mucosal diseases and keratoconjunctivitis, that can promote the recovery mechanism of living body cells, or can have bacteriostatic or bactericidal action on pathogenic microbes, on the basis of the mechanism of the natural life system. The present invention further aims to provide a therapeutic water for digestive organ mucosal diseases and keratoconjunctivitis, that may not give any damage on the digestive organ mucosal and keratoconjunctiva, being colorless, odorless and non-stimulant, and can achieve an immediate effect.

The above objects can be achieved by a therapeutic water for digestive organ mucosal diseases and keratoconjunctivitis, comprising silver-ionic water of pH 8 or more and silver ion concentration of 0.5 to 10 ppm.

In a preferred embodiment of the 7th use of the present invention, the therapeutic water may have electrical conductance of 250 to 450 $\mu$Ʊ/$cm^3$ and contain activated oxygen in a dissolved oxygen amount of 6 to 9 ppm.

The present inventors have introduced a theory of inorganic biochemistry to establish a therapeutic method entirely different from the various therapeutic methods having hitherto taken for the respective diseases of the digestive organ mucosa and keratoconjunctiva.

inventors have analyzed the rise and fall of cell in the living bodies, and thereby discovered for the first time a mechanism of artificial bacteriostatic or bactericidal action in the natural life system.

Of the cells composing a body, some live as long as it.can live once they have been completed, some repeat new formation and destruction by themselves, and there are some other various ones, but, observing the manner of the death of cells, a change often occurs firstly in the nucleus. The change occurring in the nucleus is the concentration of chromosomes, and, in contrast thereto, diffusion takes place in cytoplasms due to the inflow of water from outside. As a result of inflow of water to the cytoplasm, a structure of the membrane may be vacuolated and may be soon destroyed to lose its shape, whereupon small organs also may be thrown out and the cell come to death. In other words, it can be said that a cell, wherein a cell membrane controls the going in and out of substances, is living while the membrane functions positively, and dead when the function is lost. However, even with some damage applied to cytoplasma, the cell can repair oneself if the nucleus can sufficiently function. To analyze these mechanisms, the object can be achieved if the function of cell membranes can be artificially inactivated for a certain period, in other words,

inactivated by a special catalytic function to the composition forming the plasma membranes of cells, but, on the other hand, a prerequisite is that no adverse effect should be given to living bodies. As a result of a great number of experiments and verification having been hitherto made, the present inventors have succeeded in inactivating only the target bacteria and tissue cells having an weakened resistance due to receipt of damage, by utilizing the mechanism possessed by silver ions acting only on 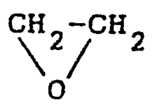, to catalyze unsaturated aliphatic acids of phospholipids constituting the cell membranes for the formation thereof into ethylene. In order to sufficiently achieve the catalytic function of silver ions in the co-presence of proteins, it is of course necessary to prevent the action of silver ions being lowered because of the complex-forming combination with proteins on the way. For that purpose, there was provided the condition of pH 8 or more under which it is considered that the protons of the lysine residual group or arginine residual group present in proteins begin to release to lower the combination ability. As a result, the silver-ionic water showed sufficient action, and, conveniently, no stimulation was brought about by even oral or intraocular administration under such a pH condition, also showing, in the case of

the gastric or duodenal ulcer, the effect of neutralizing the pH having inclined to the acidic side. Further, the $LD_{50}/ED_{50}$ to know the influence to normal human cells is desired to be as high as possible. However, since the safety value of silver ions which is obtained by experiments and verification is 500 ppm and the concentration necessary for killing the pathogenic bacteria and damaged cells, of the therapeutic water of the present invention is 0.5 to 10 ppm, there is nearly almost no harm to human bodies and also there is no problem at all even when applied in the digestive organ mucosa or keratoconjunctiva, which could not be expected at all in the conventional chemical drugs.

The therapeutic water according to the 7th use of the present invention has the same action as that of the therapeutic water according to the 4th use of the invention, and no explanation therefor will be repeated here.

The therapeutic water of the present invention contain the silver having the above action as silver ions in concentration of 0.5 to 10 ppm, more preferably 1 to 7 ppm, most preferably 2 to 4 ppm.

The silver ions in the therapeutic water of the present invention can pass through plasma cell membranes (and also cell walls) having the function of separating

and passing proteins comprising giant molecular colloidal particles from a liquid, enter the bacterial bodies, form the cell membranes into ethylene, and inactivate the function by virtue of the selective function pertaining to silver ions, i.e., the catalytic action for 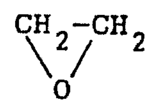. The experiments made by the present inventors revealed that the bacterial bodies once made bacteriostatic again began to regenerate and propagate themselves at the silver ion concentration of about 200 to 500 ppb, the regeneration rate was lowered with increase in the concentration, and there resulted in an almost sterilized state at the concentration of 0.5 to 10 ppm.

Also, in respect of human cells, whose safety degree of $LD_{50}/ED_{50}$ is 50 ppm for healthy normal cells, a noticeable phenomenon has been discovered, that the damaged cells such as cells attacked by pathogenic bacteria may follow substantially the same tendency as that for cell bodies. Accordingly, treatment by the therapeutic water of the present invention can inactivate pathogenic bacteria and, at the same time, break the damaged cells, and also makes it possible to eliminate the inhibition of the regeneration of tissue by such damaged cells and to achieve the regeneration with only the normal cells.

The therapeutic water of the present invention may have the pH value of pH 8 or more, more preferably pH 8.5 to 10.5, and most preferably pH 9 to 10.5. Making the therapeutic water to have the pH value of 8 or more as above, absorption of silver ions on proteins can be prevented, a lowering of the catalytic action of silver ions can be prevented even under the conditions of co-presence of proteins, and it is made possible for silver ions to freely permeate the cell membranes of target bacterial bodies and damaged cells. Also, when applied in the therapy of ulcers, the peripheral environment of the mucosa having inclined to the acidic side can be neutralized to bring about an antacidic action. Further, the silver-ionic water of the present invention may be administered under such a pH range without giving any stimulation even when administered orally or intraocularly.

The therapeutic water of the present invention may further have electrical conductance of 250 to 450 $\mu\mho/cm^3$, more preferably 350 $\mu\mho/cm^3$, and most preferably 400 $\mu\mho/cm^3$. Making the therapeutic water to have the electrical conductance of 250 to 450 $\mu\mho/cm^3$, the cell permeability of silver can be made better under the pH range of pH 8 or more, and it is made possible for the action of silver ions to be sufficiently exhibited.

In addition, the therapeutic water of the present invention may preferably contain activated oxygen in a dissolved oxygen amount of 6 to 9 ppm, more preferably 9 ppm. This is preferable to provide a desirable environment for the recovery and regeneration in the affected tissue, and very effective for bringing about hemostatic action when the affected part is bleeding. Specifically speaking, oxygen is considered to be also exasperated at the portion such as damaged tissue where the propagation and regeneration are effected, to require the oxygen in a quantity 4 times larger than the normal case. However, since actually the oxygen is supplied only in the concentration approximate to the critical value, even a slight disorder may cause a lowering of the localized partial pressure. In other words, at the portion where the number of capillaries is small and the density of vasoganglions is thin to have an increased oxygen consumption on the other hand, the localized oxygen partial pressure may be lowered to cause a hindrance to curing. Also, it may sometimes happen that the ulcers or the like may break blood vessels to cause bleeding. In general, the physiological mechanism of hemostasis is such that the coagulation inhibitory substance prostacycline which is continually released from blood vessel walls is stopped being released when blood endothelial cells are

damaged, platelets are adhered on the damaged part to make vigorous the synthesis of thromboxanthine to accelerate the formation of thrombi. In order to artificially and rapidly cause this, the present inventors, as a result of intensive studies, have found the fact that blood vessel obturation in primaturity retinopathy may be caused by unbalance between prostacycline and thromboxanthine, and, on the basis thereof, have noted that the mechanism of the formation of thrombi originates from the methabolism of prostaglandin, which is accompanied with the peroxidation reaction, thus reaching a conclusion that, for working of blood coagulation mechanism for the purpose of hemostasis, it is effective to form the conditions for the peroxidation reaction. Actually, the silver-ionic water obtained by increasing the concentration of the dissolved oxygen in the silver-ionic water and at the same time activated to increase the oxidation power, was applied on the damaged part. As a result, the regeneration of tissue was promoted, and, on the other hand, the peroxidation of the lipids in the damaged blood cell internal walls was progressed, prostacycline was artificially inhibited, thromboxanthine was released, and thus the blood coagulation mechanism was worked to achieve hemostasis.

The therapeutic water of the present invention can be produced by the aforesaid apparatus shown in Fig. 1.

The therapeutic water according to the 7th use of the present invention will be described below in greater detail with reference to some examples of diseases.

Gastric ulcer and duodenal ulcer may be usually followed by hyperacidity, although there may be some rare case of less acidity. The gastric mucosal portion may be affected because of such strong acidity to suffer ulcer. In general, this disease is considered to be readily cured by a surgery. However, since the stomach achieves a part of hematopoietic functions, various difficulties such as pernicious anemia may be caused. Also, depending on the surgery, not only the tissue damaged by ulcer but also sound tissue may be ablated. On the contrary, in the conventional medical therapy, there has been taken such a negative therapeutic method that alimentary therapy is continued so as not to give a hard load on the stomack, during which antiacids or the like are administered to recover a normal state of the secretion of acid in the stomach, relying on the mechanism of the autotherapy. Moreover, this method can be applied only in respect of primary and intermediary symptoms, and may require a very long period of time to cure. Now, the therapeutic water of the present invention, comprising silver-ionic water of pH 8 or more, silver ion concentration of 0.5 to 10 ppm,

electrical conductance of 250 to 450 µ℧/$cm^3$ and a dissolved activated-oxygen amount of 6 to 9 ppm, was orally administered to the affected part of gastric ulcer or duodenal ulcer. As a result, the environment of the affected part of the stomach or duodenum, inclined to the neutral side, was neutralized by allowing $H^+$ ions to combine with $OH^-$ ions contained in the silver-ionic water, and also the cells injured by ulcer were inactivated by being formed into ethylene by the action of silver ions acting on the unsaturated aliphatic acid residual groups in the phospholipids constituting the cell membranes, to lose the function of controlling the inflow of substance, were broken because of inflow of water from outside, and were decomposed and absorbed. This made the affected part to be in the state where only sound and normal cells are present, whereby the mechanism of regeneration with only the sound and normal cells immediately gegan to work, and the activation and propagation of the cells were so speedy that the cure period was greatly shortened to give the recovery in about 10 days. In the case the bleeding is seen due to the ulcer, it was stopped immediately after administration of the therapeutic water of the present invention, and it was recognized that the therapeutic water had a very high hemostatic effect. Moreover, in the

administration of the therapeutic water of the present invention, the therapeutic water was found to be not stimulant at all, and also found to give no chemical or physical damage against the sound and normal mucosal cells in digestive organs.

Bacterial enteritis caused by the infection with pathogenic bacteria such as Salmonella, Escherichia coli and Vivrio enteritidis is accompanied with symptoms such as heavy stomachache, diarrhea, fever and vomition, but, conventionally, any strong drugs can not be used because of the weak resistance of mucosa to chemical drugs, and has relied on the autotherapy while taking care for rest and warming. Now, the therapeutic water of the present invention, comprising silver-ionic water of pH 8 or more, silver ion concentration of 0.5 to 10 ppm, electrical conductance of 250 to 450 $\mu U/cm^3$, was orally administered to patients suffered from bacterial enteritis. As a result, silver ions reached intestinum rectum and crassum while maintaining their activities, and bactericidally or bacteriostatically acted at once on the bacteria such as Salmonella by catalyzing unsaturated aliphatic acid groups in phospholipids constituting cell membranes of the bacteria to inactivate the function of cell membranes, whereby almost perfect cure was achieved in a very short time of about 3 hours. Moreover, in the administration of

the silver-ionic water, it was found to be not stimulant at all, and also found to give no harm to the mucosal walls of digestive organs.

Epidemic keratoconjunctivitis which is a sort of infectious keratoconjunctivitis, is contagious conjunctivitis caused by adenvirus Type-8, and may epidemically prevail from spring to summer, constituting the mainstream of "Hayarime" (epidemically affected pinkeye) in our country. Symptom thereof is such that it is contracted by acute follicular conjunctivitis, showing heavy tumefaction of eyelids and edema of conjunctiva, but with less eye fat, and with complaint of lacrimation and photophobia. Conventionally, no specific medicine has been found except for ABOB eyedrops which are said to be capable of shortening the progress to a certain degree, which eyedrops, however, have a fear of causing an impediment to the function of eyes by increasing the medicinal effect, so that only those having a very weak bactericidal effect have been used, and no effective therapy has been achieved. Now, eye-washing with use of the therapeutic water of the present invention, comprising silver-ionic water of pH 8 or more, silver ion concentration of 0.5 to 10 ppm, electrical conductance of 250 to 450 $\mu U/cm^3$, was applied to patients suffered from epidemic keratoconjunctivitis. As a result, the silver

ions bactericidally or bacteriostatically acted at once on the bacterial body viruses and intermixed bacterial groups by catalyzing the cell membranes of these bacterial bodies to inactivate the function of cell membranes, whereby with immediate effectiveness, perfect cure was achieved in about a day in most part. Moreover, the eye-washing was found to be not stimulant to eyes with no other troubles at all.

Thus, the therapeutic water of the present invention can provide an effective and harmless therapeutic method based on the physiological mechanism, in respect of the diseases such as gastric ulcer, duodenal ulcer, bacterial enteritis and epidemic keratoconjunctivitis against which no effective therapeutic method has not been found because of the low resistance of the digestive organ mucosa and keratoconjunctiva and for other reasons. It, however, will be apparent that the present therapeutic water can have the similar effect to effectively cope with other diseases than those mentioned here in respect of the digestive organ mucosa and keratoconjunctiva of eyes.

[Examples of the 7th use]

Example 7-1 Hemorrhagic gastritis

Symptom:

Complaints at the epigustrium. Diagnosis using an endospcopy revealed punctate erosive bleeding on the

surface of gastric mucosa, and the failure was judged to have occurred only at the mucosal layer.

Treatment:

Silver ionic water of pH 10, silver ion concentration of 2 ppm, electrical conductance of 400 µ℧ /$cm^3$ and a dissolved oxygen amount of 9 ppm was orally administered in an amount of 2.1 lit. in a weak (100 cc, three times a day).

Therapeutic effect:

A feeling of oppression was removed on the second day after washing by oral administration, and the inconstant complaints were eliminated after 4 days.

After 10 days, the endoscopic observation revealed that the mucosal layer turned normal with no bleeding seen and with perfect cure.

Example 7-2 Acute gastric ulcer

Symptom:

Ache very frequently developed at the stomach, accompanied with hematochezia. As a result of diagnosis using an endospcopy, there was observed three ulcerated portions of 1 cm to 1.5 cm in diameter each. One of them was observed to be a rupture reaching the muscular layer of mucosa, and two of them were observed to be ruptures reaching the intrinsic muscular layer.

Treatment:

Using 2 lit. of silver ionic water of pH 10.5, silver ion concentration of 2 ppm, electrical conductance of 350 $\mu \mho/cm^3$ and a dissolved oxygen amount of 9 ppm, gastrolavage was carried out through oral administration of the water, and further 0.2 lit. of the water were orally administered for 7 days at intervals of 8 hours.

Therapeutic effect:

The ache stopped in one hour after the first treatment, and endoscopic diagnosis after 4 days revealed that the ruptured portions were covered with regenerated mucosa, and formation of fibrous tissue proceeded. Endoscopic observation of the stomach after further one week diagnosed that the mechanism of perfect cure completed.

Example 7-3 Duodenal retrobulb ulcer

Symptom:

Heavy ache 2 to 4 hours after diet, accompanied with hematochezia. Since indirect X-ray diagnosis revealed a mechanical constriction and prediagnozed that there would be an ulcer on the opposite side, endoscopic examination was further carried out. As a result, an ulcer was found at a superior corner of duodenum on the side near to the anus.

Treatment:

Silver ionic water of pH 10, silver ion

concentration of 2 ppm, electrical conductance of 350 $\mu \mho/cm^3$ and a dissolved oxygen amount of 9 ppm was continually orally administered in an amount of 4 lit., and oral administration of 2 lit. of the water was repeated for further 10 days.

Therapeutic effect:

After the treatment on the first day, the ache was softened, and turned almost acheless from the second day. A feeling of complaint stopped at the forth day. X-ray diagnosis after a week revealed the loosening of the constriction, and endoscopic observation after 10 days diagnozed that the ulcer disappeared and regenerated mucosa appeared.

Example 7-4    Infectious diarrhea

Symptom:

Acute diarrhea and fever were caused, and glairy hematochezia was observed, accompanied with abdominalgia along with vomic feeling, vomition and inappetence. Clinical observation threw doubt on enterisis vivriosis, Salmonella enterisis or the like, and an emergent treatment was required before the outcome of bacterial culture.

Treatment:

Silver ionic water of pH 10, silver ion concentration of 2 ppm, electrical conductance of 400

u /cm$^3$ and a dissolved oxygen amount of 9 ppm was continually orally administered in an amount of 200 cc.

Therapeutic effect:

The vomic feeling and vomition were went over two hours after the oral administration, and, after 3 hours, the fever and ache were also removed and the diarrheal symptom was also disappeared. No infectious diarrheal symptom occurred thereafter, with perfect cure.

Example 7-5 Pyeletis

Symptom:

Fever and gross hematuria were observed, and, as a result of IVP applied, pyeletic signs such as kidney turned oval or clavate were observed to cause an imperfect vesicourethral canal countercurrent phenomenon.

Treatment:

Silver ionic water of pH 10, silver ion concentration of 2 ppm, electrical conductance of 400 µU /cm$^3$ and a dissolved oxygen amount of 9 ppm was continually orally administered in an amount of 5 lit. over a period of 24 hours.

Therapeutic effect:

Even though a sulfa drug was administered for a long period without perfect cure, application of the present therapy resulted in a descent of fever after 2 hours, a

stop of tremor after 3 hours, and, after 5 hours, made it possible for the patient to rise from a bed.

Since the subjective symptom was disappeared from the patient after 24 hours, IVP was again applied to reveal that the sign of urinary tract infectious disease including pyrogenic attack was perfectly disappeared.

Example 7-6 Conjunctivitis

Symptom:

Contracted by acute follicular conjunctivitis, showing heavy tumefaction of eyelids and edema of conjunctiva, but with less eye fat, and with complaint of lacrimation, accompanied with edema of a lymph gland in front of the ear and tenderness observed. Diagonized as an infectious disease caused by adenovirus Type-8.

Treatment:

Silver ionic water of pH 9, silver ion concentration of 1 ppm, electrical conductance of 350 $\mu \mho/cm^3$ and a dissolved oxygen amount of 9 ppm was eyedropped 4 times a day, and this was carried out continually for 7 days.

Therapeutic effect:

Lacrimation and photophobia were stopped after the first eyedropping, the feeling of tumefaction was removed on the second day, the edema of conjunctiva disappeared perfectly on the third day, and the subjective symptoms such as the pressure at the lymph gland were removed on

the seventh day with perfect cure.

As described in the foregoing, the present invention is a therapeutic water for digestive organ mucosal diseases and keratoconjunctivitis, comprising silver-ionic water of pH 8 or more and silver ion concentration of 0.5 to 10 ppm, that can promote the mechanism of the recovery of living body cells or can bacteriostatically or bactericidally act on pathogenic bacteria according to the function or mechanism entirely different from the conventional chemotherapic agents. On the other hand, it is an excellent therapeutic water that may not give any damage on the sound and normal digestive organ mucosa and keratoconjunctiva tissue, being colorless, odorless and non-stimulative, and can achieve an immediate effect, and can be preferably used for the therapy of digestive mucosal diseases such as gastric ulcer, duodenal ulcer and bacterial enteritis, and pyeletis, and also for keratoconjunctivitis such as epidemic keratoconjunctivitis and acute hemorrhagic conjunctivitis.

WHAT IS CLAIMED IS:

1. A silver-ionic water having the pH of not more than 5 or not less than 8 and a silver ion concentration of 0.03 to 10 ppm.

2. The silver-ionic water of Claim 1, further having an electrical conductance of 250 to 2,000 $\mu℧/cm^3$.

3. The silver-ionic water of Claim 1 for use as a bacteriostatic water comprising a silver-ionic water of pH 5 or less or pH 8 or more and a silver ion concentration of 200 to 2,000 ppb.

4. The silver-ionic water of Claim 3, wherein said bacteriostatic water comprises a silver-ionic water of pH 5 or less and an electrical conductance of 350 to 2,000 $\mu℧/cm^3$.

5. A method for bacteriostatic treatment comprising spraying a silver-ionic water of pH 5 or less and a silver ion concentration of 200 to 2,000 ppb in the form of a mist.

6. The bacteriostatic method of Claim 5, wherein said silver-ionic water has an electrical conductance of 350 to 2,000 $\mu℧/cm^3$.

7. The silver ionic water of Claim 1 for use as a growth promotive acidic water for plant cultivation, comprising a silver-ionic water of pH 5 or less and a silver

ion concentration of 30 to 2,000 ppb.

8. The silver-ionic water of Claim 7, wherein said growth promotive acidic water for plant cultivation comprises said silver-ionic water further having an electrical conductance of 350 to 2,000 $\mu\mho/cm^3$ and a dissolved oxygen content of 12 to 30 ppm.

9. The silver ionic water of Claim 1, for use as a growth promotive alkaline water for plant cultivation, comprising a silver-ionic water of pH 8 or more and a silver ion concentration of 30 to 2,000 ppb.

10. The silver-ionic water of Claim 9, wherein said growth promotive alkaline water for plant cultivation comprises said silver-ionic water further having an electrical conductance of 250 to 450 $\mu\mho/cm^3$.

11. The silver ionic water of Claim 1, for use as a therapeutic water for pathogenobacterial skin diseases and external cavity mucosal diseases, comprising a silver-ionic water of pH 5 or less and a silver ion concentration of 0.5 to 10 ppm.

12. The silver-ionic water of Claim 11, wherein said therapeutic water comprises said silver-ionic water further having an electrical conductance of 350 to 2,000 $\mu\mho/cm^3$ and a dissolved oxygen amount of 12 to 30 ppm.

13. The silver ionic water of Claim 1, for use as a wound-curative water, comprising the silver-ionic water of

pH 5 or less, a silver ion concentration of 0.5 to 10 ppm and electrical conductance of 250 to 2,000 $\mu℧/cm^3$

14. The silver-ionic water of Claim 13, wherein said therapeutic water comprises said silver-ionic water further containing activated oxygen in a dissolved oxygen content of 12 to 30 ppm.

15. The silver ionic water of Claim 1, for use as a disinfectant water, comprising a silver-ionic water of pH 5 or less and a silver ion concentration of 0.5 to 10 ppm.

16. The silver-ionic water of Claim 15, wherein said disinfectant water comprises silver-ionic water further having an electrical conductance of 350 to 2,000 $\mu℧/cm^3$.

17. The silver ionic water of Claim 1, for use as a therapeutic water for digestive organ mucosal diseases and keratoconjunctivitis, comprising a silver-ionic water of pH 8 or more and a silver ion concentration of 0.5 to 10 ppm.

18. The silver-ionic water of Claim 17, wherein said therapeutic water comprises silver-ionic water further having an electrical conductance of 350 to 2,000 $\mu℧/cm^3$ and containing activated oxygen in a dissolved oxygen content of 6 to 9 ppm.

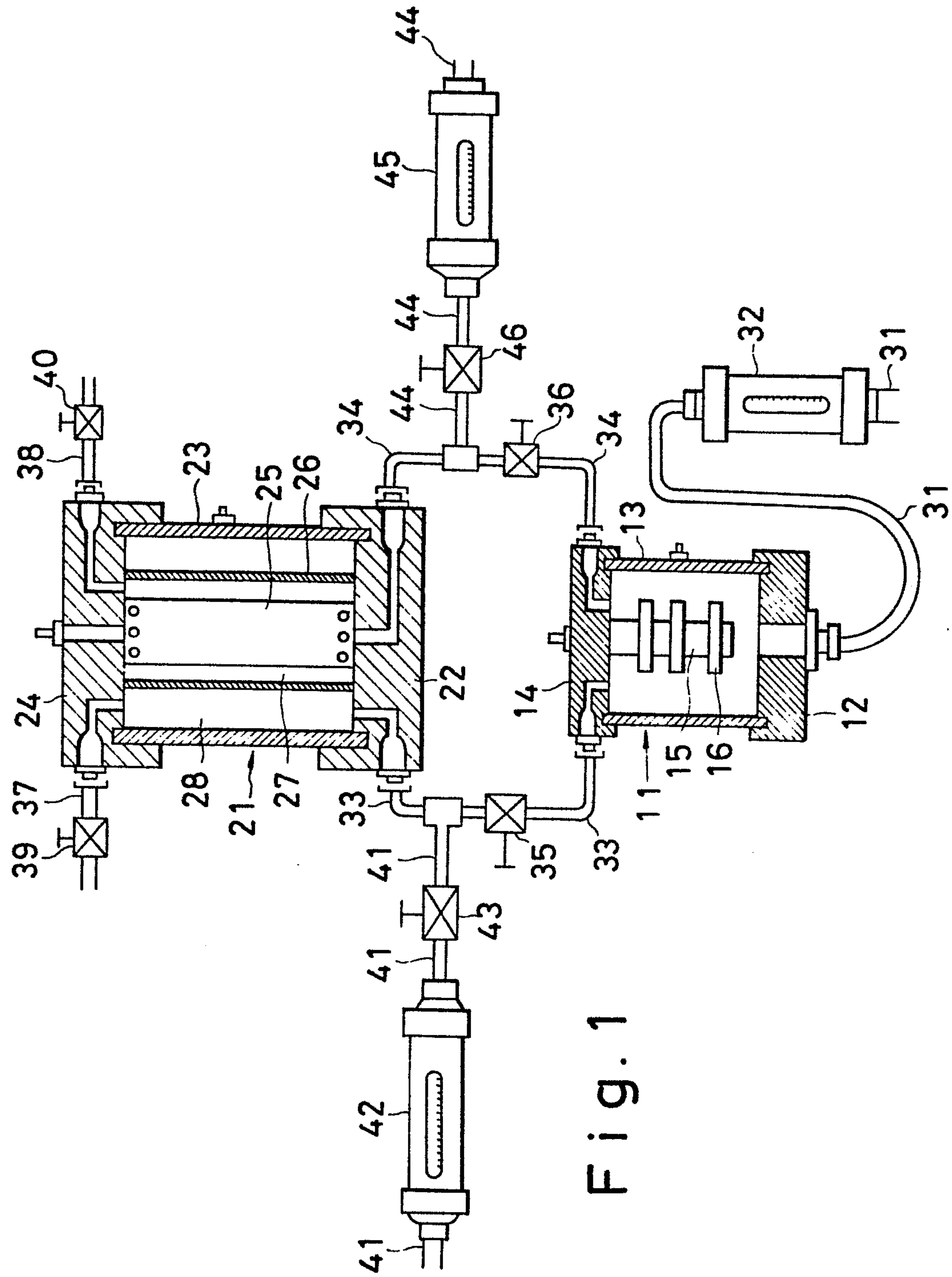
44
45
44
46
44
34
36
34
40
38
23
25
26
32
31
31
13
14
12
15
16
11
22
24
28
27
21
33
35
33
37
39
41
43
41
42
41

Fig. 1

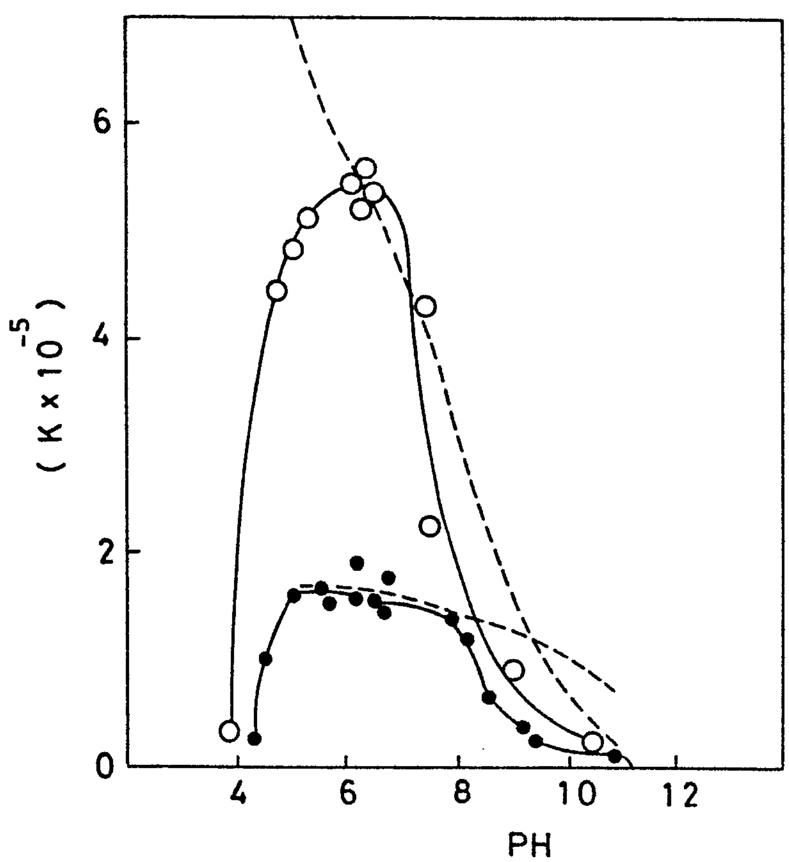


Fig.2

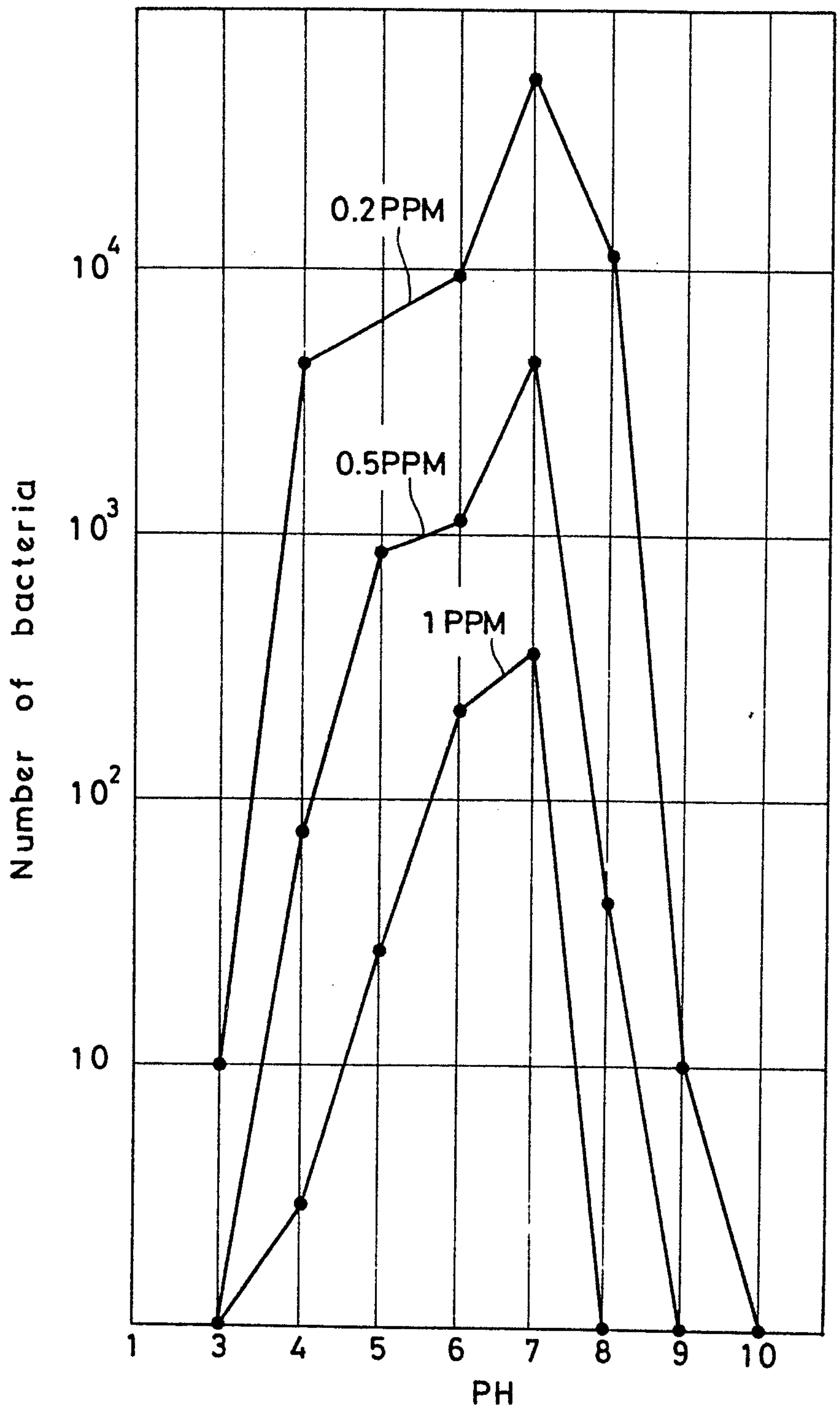

0.2PPM
0.5PPM
1PPM
$10^4$
$10^3$
$10^2$
10
Number of bacteria
1
3
4
5
6
7
8
9
10
PH


Fig. 3

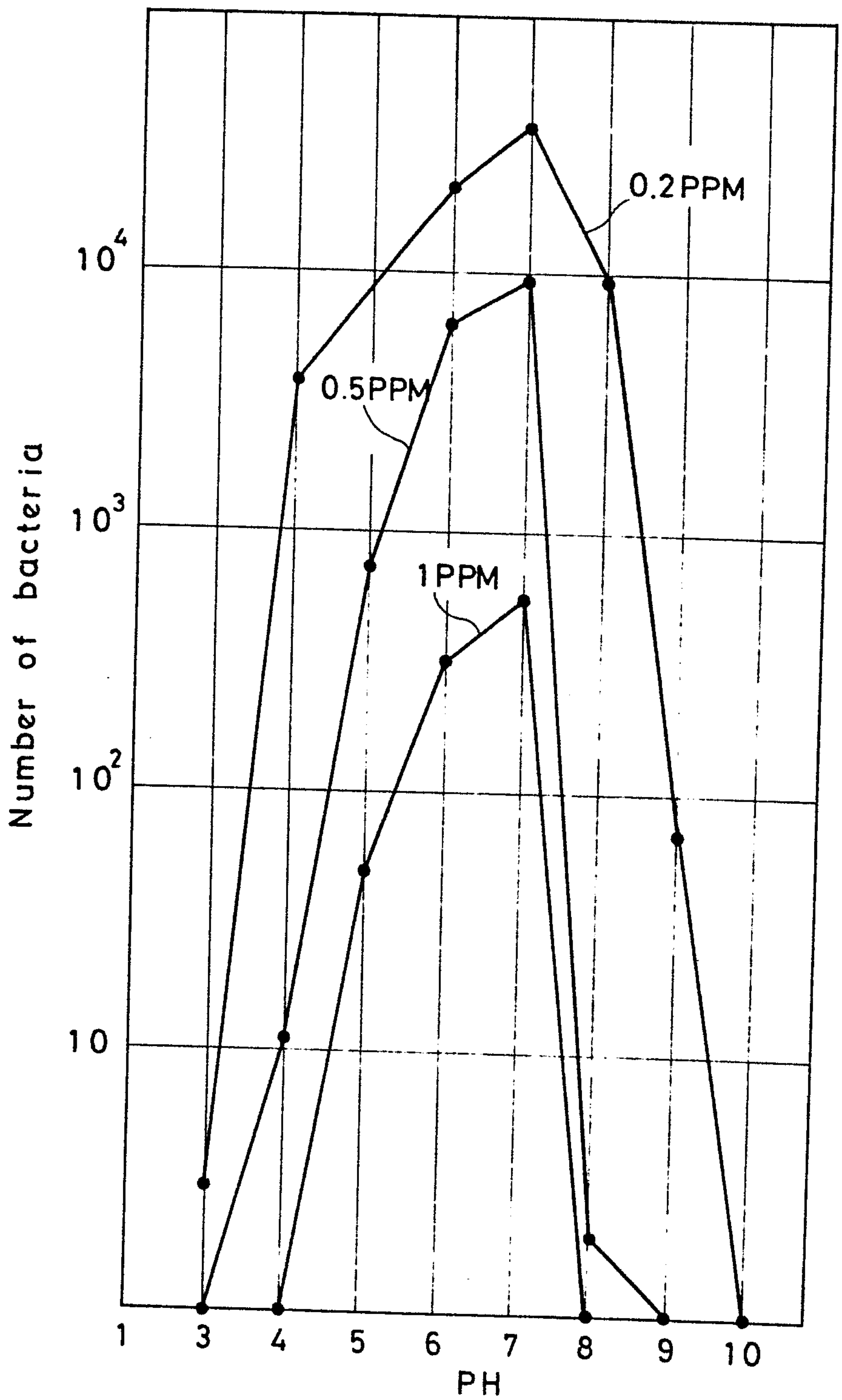

0.2PPM
0.5PPM
1PPM
$10^4$
$10^3$
$10^2$
10
Number of bacteria
1
3
4
5
6
7
8
9
10
PH


Fig. 4

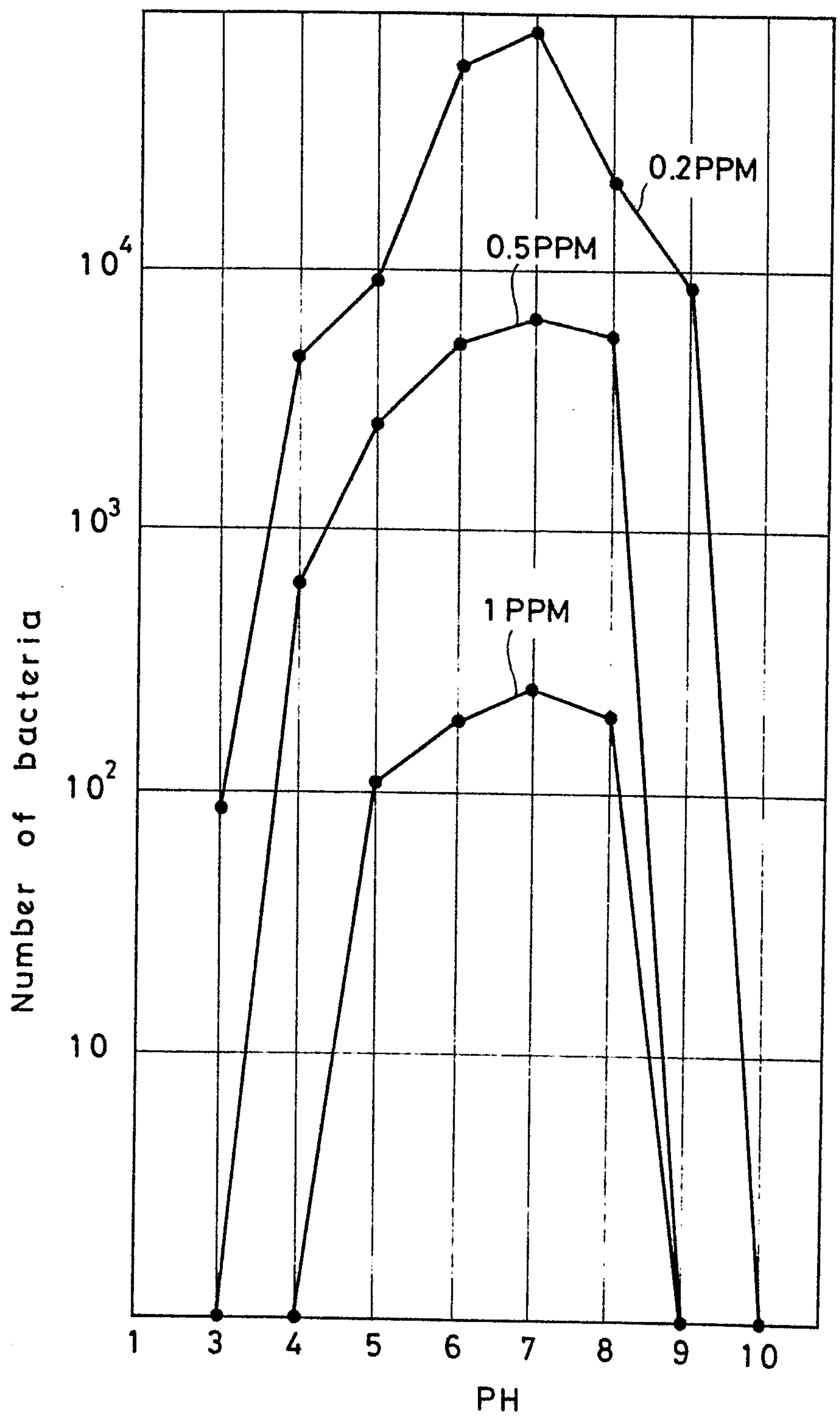
0.2PPM
0.5PPM
1 PPM
$10^4$
$10^3$
$10^2$
10
Number of bacteria
1
3
4
5
6
7
8
9
10
PH

Fig.5

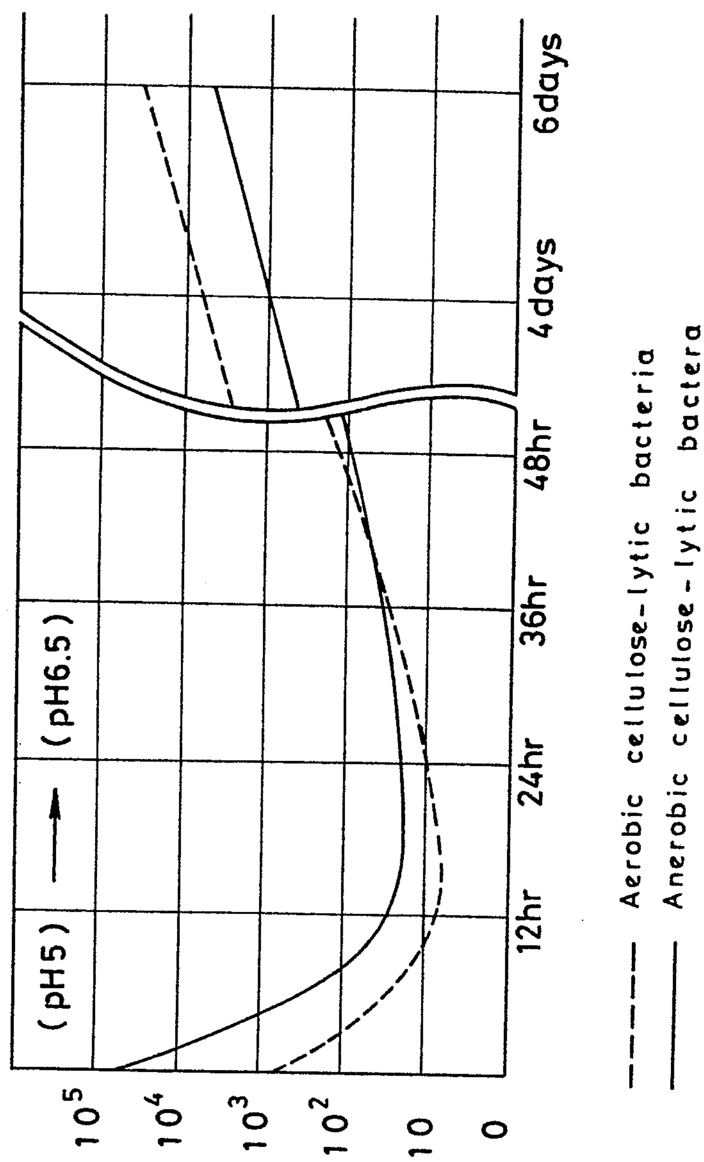
(pH5) → (pH6.5)
$10^5$
$10^4$
$10^3$
$10^2$
10
0
12hr
24hr
36hr
48hr
4days
6days
Aerobic cellulose-lytic bacteria
Anerobic cellulose-lytic bacteria

Fig.6

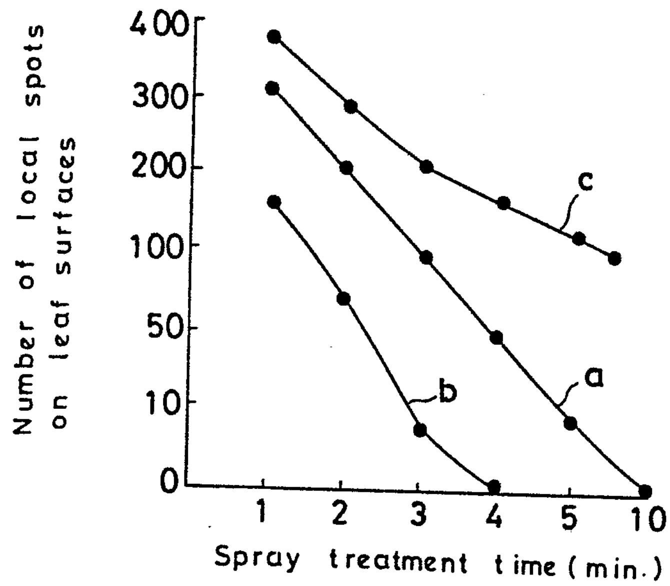
Number of local spots on leaf surfaces
400
300
200
100
50
10
0
c
a
b
1
2
3
4
5
10
Spray treatment time (min.)

Fig. 7

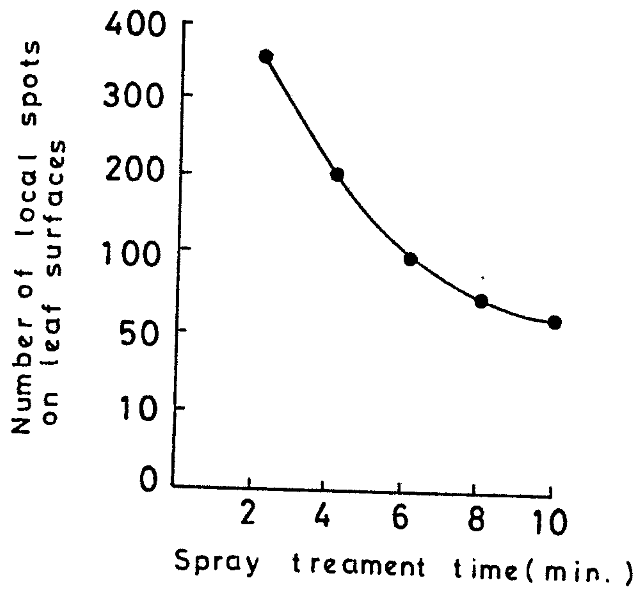
Number of local spots on leaf surfaces
400
300
200
100
50
10
0
2
4
6
8
10
Spray treament time (min.)

Fig. 8